# EUROPÄISCHE PATENTSCHRIFT

(11) **EP 1 697 379 B1**
(45) Veröffentlichungstag und Bekanntmachung des Hinweises auf die Patenterteilung: **25.03.2009**
(21) Anmeldenummer: 04804137.0
(22) Anmeldetag: 21.12.2004
(51) Int. Cl.: C07D 491/10, C07D 495/10, C07D 493/10, A61P 25/28, A61K 31/382, A61K 31/404, A61K 31/352

(54) **SPIROCYCLISCHE CYCLOHEXAN-DERIVATE**
SPIROCYCLIC CYCLOHEXANE DERIVATIVES
DERIVES SPIROCYCLIQUES DE CYCLOHEXANE

(30) Priorität: 23.12.2003 DE 10360793
(43) Veröffentlichungstag der Anmeldung: 06.09.2006
(73) Patentinhaber: Grünenthal GmbH, 52078 Aachen (DE)
(72) Erfinder: HINZE, Claudia, 52066 Aachen (DE); SUNDERMANN, Bernd, 52074 Aachen (DE); SCHICK, Hans, DE-13086 Berlin (DE); HENKEL, Birgitta, 12555 Berlin (DE)
(74) Vertreter: Bülle, Jan
(86) Internationale Anmeldenummer: PCT/EP2004/014540
(87) Internationale Veröffentlichungsnummer: WO 2005/063769

(56) Entgegenhaltungen:
- EP-A- 1 142 587
- WO-A-20/04043967

## Beschreibung

Die vorliegende Erfindung betrifft spirocyclische Cyclohexan-Derivate, Verfahren zu deren Herstellung, Arzneimittel enthaltend diese Verbindungen und die Verwendung von spirocyclischen Cyclohexan-Derivaten zur Herstellung von Arzneimitteln.

Die Behandlung chronischer und nichtchronischer Schmerzzustände hat in der Medizin eine große Bedeutung. Es besteht ein weltweiter Bedarf an gut wirksamen Schmerztherapien. Der dringende Handlungsbedarf für eine patientengerechte und zielorientierte Behandlung chronischer und nicht chronischer Schmerzzustände, wobei hierunter die erfolgreiche und zufriedenstellende Schmerzbehandlung für den Patienten zu verstehen ist, dokumentiert sich in der großen Anzahl von wissenschaftlichen Arbeiten, die auf dem Gebiet der angewandten Analgetik bzw. der Grundlagenforschung zur Nociception in letzter Zeit erschienen sind.

Klassische µ-Opioide wie Morphin sind bei der Therapie starker bis stärkster Schmerzen gut wirksam und von größter Bedeutung für die Schmerztherapie. Es kann jedoch von Vorteil sein, wenn neben dem µ-Opioid-Rezeptor auch andere Opioid-Rezeptoren (δ, κ, ORL-1) beeinflusst werden, da die reinen µ-Opioide auch unerwünschte Nebenwirkungen wie Obstipation und Atemdepression aufweisen, aber auch zu Abhängigkeit führen können. Auch die Opioid-Rezeptoren δ, κ und ORL-1 sind am Schmerzgeschehen beteiligt (Opioids: Introduction, S. 127-150, Further Opioid Receptors, 455-476 in: Analgesics - From Chemistry and Pharmacology to Clinical Application, Wiley VCH 2002).

Der ORL1-Rezeptor ist außerdem noch an der Regulation weiterer physiologischer und pathophysiologischer Prozesse beteiligt. Hierzu gehören unter anderem Lernen und Gedächtnisbildung (Manabe et al., Nature, 394, 1997, S. 577-581), Hörvermögen (Nishi et al., EMBO J., 16, 1997, S. 1858-1864) sowie zahlreiche weitere Prozesse. In einem Übersichtsartikel von Calo et al. (Br.J. Pharmacol., 129, 2000, 1261 -1283) wird ein Überblick über die Indikationen oder biologischen Vorgänge gegeben, in denen der ORL1-Rezeptor eine Rolle spielt oder mit hoher Wahrscheinlichkeit spielen könnte. Genannt werden u.a.: Analgesie, Stimulation und Regulation der Nahrungsaufnahme, Einfluß auf µ-Agonisten wie Morphin, Behandlung von Entzugserscheinungen, Reduzierung des Suchtpotentials von Opioiden, Anxiolyse, Modulation der Bewegungsaktivität, Gedächtnis-Störungen, Epilepsie; Modulation der Neurotransmitter-Ausschüttung, insbesondere von Glutamat, Serotonin und Dopamin, und damit neurodegenerative Erkrankungen; Beeinflußung des cardiovaskulären Systems, Auslösung einer Erektion, Diurese, Antinatriurese, Elektrolyt-Haushalt, arterieller Blutdruck, Wasserspeicher-Krankheiten, intestinale Motilität (Diarrhoe), relaxierende Effekte auf die Atemwege, Mikturations Reflex (Harninkontinenz). Weiter wird die Verwendung von Agonisten und Antagonisten als Anoretika, Analgetika (auch in Coadministration mit Opioiden) oder Nootropika diskutiert.

EP-A-1 142 587 offenbart Opioid-Rezeptor Agonisten mit einer Spiro-imidazolino-Piperidine Komponente.

Aufgabe der vorliegenden Erfindung war es, Arzneimittel zur Verfügung zu stellen, die auf das Opioid-Rezeptor-System wirken und damit für Arzneimittel insbesondere zur Behandlung der verschiedenen mit diesem System nach dem Stand der Technik in Verbindung stehenden Krankeiten bzw. zum Einsatz in den dort genannten Indikationen geeignet sind.

Gegenstand der Erfindung sind daher spirocyclische Cyclohexan-Derivate der allgemeinen Formel I, 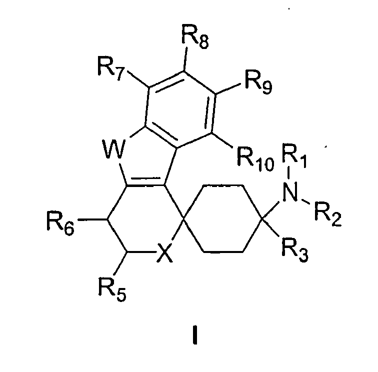 , worin
R¹ und R², unabhängig voneinander für H; CHO; C₁₋₅-Alkyl jeweils gesättigt oder ungesättigt, verzweigt oder unverzweigt, einfach oder mehrfach substituiert oder unsubstituiert; C₃₋₈-Cycloalkyl, jeweils gesättigt oder ungesättigt, einfach oder mehrfach substituiert oder unsubstituiert; oder über C₁₋₃-Alkyl gebundenes Aryl, C₃₋₈-Cycloalkyl oder Heteroaryl, jeweils einfach oder mehrfach substituiert oder unsubstituiert, stehen;
oder die Reste R¹ und R² zusammen für CH₂CH₂OCH₂CH₂, CH₂CH₂NR¹¹ CH₂CH₂ oder (CH₂)₃₋₆ stehen,
   wobei R¹¹ H; C₁₋₅-Alkyl, jeweils gesättigt oder ungesättigt, verzweigt oder unverzweigt, einfach oder mehrfach substituiert oder unsubstituiert; C₃₋₈-Cycloalkyl, jeweils gesättigt oder ungesättigt, einfach oder mehrfach substituiert oder unsubstituiert; Aryl-, oder Heteroaryl, jeweils einfach oder mehrfach substituiert oder unsubstituiert; oder über C₁₋₃-Alkyl gebundenes Aryl, C₃₋₈-Cycloalkyl oder Heteroaryl, jeweils einfach oder mehrfach substituiert oder unsubstituiert, bedeutet;
R³ für C₁₋₅-Alkyl, jeweils gesättigt oder ungesättigt, verzweigt oder unverzweigt, einfach oder mehrfach substituiert oder unsubstituiert; C₃₋₈-Cycloalkyl, jeweils gesättigt oder ungesättigt, einfach oder mehrfach substituiert oder unsubstituiert; Aryl oder Heteroaryl, jeweils unsubstituiert oder einfach oder mehrfach substituiert; über C₁₋₃-Alkyl-Gruppe gebundenes Aryl, Heteroaryl oder C₃₋₈-Cycloalkyl, jeweils unsubstituiert oder einfach oder mehrfach substituiert, steht;
W für NR⁴, O oder S steht
   und
   R⁴ für H; C₁₋₅-Alkyl, gesättigt oder ungesättigt, verzweigt oder unverzweigt, unsubstituiert oder einfach oder mehrfach substituiert; Aryl, oder Heteroaryl, jeweils substituiert oder unsubstituiert; über eine C₁₋₃-Alkyl-Gruppe gebundenes Aryl, Heteroaryl oder Cycloalkyl, jeweils einfach oder mehrfach substituiert oder unsubstituiert; COR¹² ; SO₂R¹² steht,
      wobei R¹² H; C₁₋₅-Alkyl, jeweils gesättigt oder ungesättigt, verzweigt oder unverzweigt, einfach oder mehrfach substituiert oder unsubstituiert; C₃₋₈-Cycloalkyl, jeweils gesättigt oder ungesättigt, einfach oder mehrfach substituiert oder unsubstituiert; Aryl-, oder Heteroaryl, jeweils einfach oder mehrfach substituiert oder unsubstituiert; oder über C₁₋₃-Alkyl gebundenes Aryl, C₃₋₈-Cycloalkyl oder Heteroaryl, jeweils einfach oder mehrfach substituiert oder unsubstituiert; OR¹³; NR¹⁴R¹⁵ bedeutet;
R⁵ für =O; H; COOR¹³, CONR¹³, OR¹³; C₁₋₅-Alkyl, gesättigt oder ungesättigt, verzweigt oder unverzweigt, unsubstituiert oder einfach oder mehrfach substituiert; C₃₋₈-Cycloalkyl, gesättigt oder ungesättigt, unsubstituiert oder einfach oder mehrfach substituiert; Aryl-, oder Heteroaryl, unsubstituiert oder einfach oder mehrfach substituiert; oder über C₁₋₃-Alkyl gebundenes Aryl, C₃₋₈-Cycloalkyl oder Heteroaryl, unsubstituiert oder einfach oder mehrfach substituiert, steht; .
R⁶ für H; F, Cl, NO₂, CF₃, OR¹³, SR¹³, SO₂R¹³, SO₂OR¹³, CN, COOR¹³, NR¹⁴R¹⁵; C₁₋₅-Alkyl, gesättigt oder ungesättigt, verzweigt oder unverzweigt, unsubstituiert oder einfach oder mehrfach substituiert; C₃₋₈-Cycloalkyl, gesättigt oder ungesättigt, unsubstituiert oder einfach oder mehrfach substituiert; Aryl-, oder Heteroaryl, unsubstituiert oder einfach oder mehrfach substituiert; oder über C₁₋₃-Alkyl gebundenes Aryl, C₃₋₈-Cycloalkyl oder Heteroaryl, unsubstituiert oder einfach oder mehrfach substituiert, steht;
oder R⁵ und R⁶ gemeinsam (CH₂)ₙ mit n = 2, 3, 4, 5 oder 6 bedeuten, wobei einzelne Wasserstoffatome auch durch F, Cl, Br, I, NO₂, CF₃, OR¹³, CN oder C₁₋₅-Alkyl ersetzt sein können;
R⁷, R⁸, R⁹ und R¹⁰ unabhängig voneinander für
H, F, Cl, Br, I, NO₂, CF₃, OR¹³, SR¹³, SO₂R¹³, SO₂OR¹³, CN, COOR¹³, NR¹⁴R¹⁵; C₁₋₅-Alkyl, C₃₋₈-Cycloalkyl, unsubstituiert oder einfach oder mehrfach substituiert; Aryl-, oder Heteroaryl, unsubstituiert oder einfach oder mehrfach substituiert; oder über C₁₋₃-Alkyl gebundenes Aryl, C₃₋₈-Cycloalkyl oder Heteroaryl, unsubstituiert oder einfach oder mehrfach substituiert, stehen;
   wobei R¹³ H; C₁₋₅-Alkyl jeweils gesättigt oder ungesättigt, verzweigt oder unverzweigt, unsubstituiert oder einfach oder mehrfach substituiert; C₃₋₈-Cycloalkyl, jeweils gesättigt oder ungesättigt, unsubstituiert oder einfach oder mehrfach substituiert; Aryl-, oder Heteroaryl, unsubstituiert oder einfach oder mehrfach substituiert; oder über C₁₋₃-Alkyl gebundenes Aryl, C₃₋₈-Cycloalkyl oder Heteroaryl, unsubstituiert oder einfach oder mehrfach substituiert, bedeutet;
   R¹⁴ und R¹⁵ unabhängig voneinander H; C₁₋₅-Alkyl , jeweils gesättigt oder ungesättigt, verzweigt oder unverzweigt, unsubstituiert oder einfach oder mehrfach substituiert; oder C₃₋₈ -Cycloalkyl, jeweils gesättigt oder ungesättigt, unsubstituiert oder einfach oder mehrfach substituiert; Aryl-, oder Heteroaryl, unsubstituiert oder einfach oder mehrfach substituiert; oder über C₁-₃-Alkyl gebundenes Aryl, C₃₋₈-Cycloalkyl oder Heteroaryl, unsubstituiert oder einfach oder mehrfach substituiert, bedeuten;
      oder R¹⁴ und R¹⁵ zusammen CH₂CH₂OCH₂CH₂, CH₂CH₂NR¹⁶CH₂CH₂ oder (CH₂)₃₋₆ bilden,
      wobei R¹⁶ H; C₁₋₅-Alkyl gesättigt oder ungesättigt, verzweigt oder unverzweigt, unsubstituiert oder einfach oder mehrfach substituiert, bedeutet;
X für O, S, SO, SO₂ oder NR¹⁷ steht;
   R¹⁷ für H; C₁₋₅-Alkyl, gesättigt oder ungesättigt, verzweigt oder unverzweigt; COR¹² oder SO₂R¹² steht,
   in Form des Razemats; der Enantiomere, Diastereomere, Mischungen der Enantiomere oder Diastereomere oder eines einzelnen Enantiomers oder Diastereomers; der Basen und/oder Salze physiologisch verträglicher Säuren oder Kationen.

Bei der Zusammenfassung verschiedener Reste, beispielsweise R⁷, R⁸, R⁹ und R¹⁰ sowie der Zusammenfassung von Resten an deren Substituenten, wie z. B. OR¹³, SR¹³, SO₂R¹³ oder COOR¹³, kann ein Substituent, z.B. R¹³, für zwei oder mehrere Reste, beispielsweise R⁷, R⁸, R⁹ und R¹⁰, innerhalb einer Substanz unterschiedliche.Bedeutungen annehmen.

Die erfindungsgemäßen Verbindungen zeigen gute Bindung an den µ-Rezeptor, aber auch an andere Opioidrezeptoren. Überraschenderweise zeigte es sich, dass die Substanzen auch eine Affinität zur Bindungsstelle 2 des Natriumkanals (BTX-Bindung) aufweisen.

Dadurch ist die Verbindurigsklasse der allgemeinen Formel 1 auch zur Verwendung als Lokalanästetikum geeignet.

Die Ausdrücke "C₁₋₅-Alkyl" und "C₁₋₃-Alkyl" umfassen im Sinne dieser Erfindung acyclische gesättigte oder ungesättigte Kohlenwasserstoffreste, die verzweigt- oder geradkettig sowie unsubstituiert oder ein- oder mehrfach substituiert sein können, mit 1, 2, 3, 4 oder 5 C-Atomen bzw. 1, 2 oder 3 C-Atomen, d.h. C₁₋₅-Alkanyle, C₂₋₅-Alkenyle und C₂₋₅-Alkinyle bzw. C₁₋₃-Alkanyle, C₂₋₃-Alkenyle und C₂₋₃-Alkinyle. Dabei weisen Alkenyle mindestens eine C-C-Doppelbindung und Alkinyle mindestens eine C-C-Dreifachbindung auf. Vorteilhaft ist Alkyl aus der Gruppe ausgewählt, die Methyl, Ethyl, n-Propyl, 2-Propyl, n-Butyl, iso-Butyl, sec.-Butyl, tert.-Butyl, n-Pentyl, iso-Pentyl, neo-Pentyl, n-Hexyl, 2-Hexyl; Ethylenyl (Vinyl), Ethinyl, Propenyl (-CH₂CH=CH₂, -CH=CH-CH₃, -C(=CH₂)-CH₃), Propinyl (-CH-C=CH, - C≡C-CH₃), 1,1-Dimethylethyl, 1,1-Dimethylpropyl, Butenyl, Butinyl, Pentenyl und Pentinyl, umfaßt.

Der Ausdruck "Cycloalkyl" oder "C₃₋₈-Cycloalkyl" bedeutet für die Zwecke dieser Erfindung cyclische Kohlenwasserstoffe mit 3, 4, 5, 6, 7 oder 8 Kohlenstoffatomen, wobei die Kohlenwasserstoffe gesättigt oder ungesättigt (aber nicht aromatisch), unsubstituiert oder ein- oder mehrfach substituiert sein können. In Bezug auf Cycloalkyl umfasst der Begriff auch gesättigte oder ungesättigte (aber nicht aromatische) Cycloalkyle, in denen ein oder zwei Kohlenstoffatome durch ein Heteroatom S, N oder O ersetzt sind. Vorteilhaft ist C₃₋₈-Cycloalkyl aus der Gruppe ausgewählt, die Cyclopropyl, Cyclobutyl, Cyclopentyl, Cyclohexyl, Cycloheptyl, Cyclooctyl, Cyclopentenyl, Cyclohexenyl, Cycloheptenyl und Cyclooctenyl, aber auch Tetrahydropyranyl, Dioxanyl, Dioxolanyl, Morpholinyl, Piperidinyl, Piperazinyl, Pyrazolinonyl und Pyrrolidinyl enthält.

Unter dem Begriff (CH₂)₃₋₆ ist -CH₂-CH₂-CH₂-, -CH₂-CH₂-CH₂-CH₂-, -CH₂-CH₂-CH₂-CH₂-CH₂- und CH₂-CH₂-CH₂-CH₂-CH₂-CH₂- zu verstehen.

Der Ausdruck "Aryl" bedeutet im Sinne dieser Erfindung carbocyclische Ringsysteme mit mindestens einem aromatischen Ring, aber ohne Heteroatome in nur einem der Ringe, u.a: Phenyle, Naphthyle und Phenanthrenyle, Fluoranthenyle, Fluorenyle, Indanyle und Tetralinyle. Die Aryl-Reste können auch mit weiteren gesättigten, (partiell) ungesättigten oder aromatischen Ringsystemen kondensiert sein. Jeder Aryl-Rest kann unsubstituiert oder einfach oder mehrfach substituiert vorliegen, wobei die Aryl-Substituenten gleich oder verschieden und in jeder beliebigen und möglichen Position des Aryls sein können. Besonders vorteilhaft sind Phenyl- oder Naphthyl-Reste.

Der Ausdruck "Heteroaryl" steht für einen 5-, 6- oder 7-gliedrigen cyclischen aromatischen Rest, der mindestens 1, ggf. auch 2, 3, 4 oder 5 Heteroatome, enthält, wobei die Heteroatome gleich oder verschieden sind und der Heterocyclus unsubstituiert oder ein- oder mehrfach substituiert sein kann; im Falle der Substitution am Heterocyclus können die Substituenten gleich oder verschieden sein und in jeder beliebigen und möglichen Position des Heteroaryls sein. Der Heterocyclus kann auch Teil eines bi- oder polycyclischen Systems sein. Bevorzugte Heteroatome sind Stickstoff, Sauerstoff und Schwefel. Es ist bevorzugt, daß der Heteroaryl-Rest ausgewählt ist aus der Gruppe, die Pyrrolyl, Indolyl, Furyl (Furanyl), Benzofuranyl, Thienyl (Thiophenyl), Benzothienyl, Benzothiadiazolyl, Benzothiazolyl, Benzotriazolyl, Benzodioxolanyl, Benzodioxanyl, Phtalazinyl, Pyrazolyl, Imidazolyl, Thiazolyl, Oxazolyl, Isoxazoyl, Pyridinyl, Pyridazinyl, Pyrimidinyl, Pyrazinyl, Pyranyl, Indazolyl, Purinyl, Indolizinyl, Chinolinyl, Isochinolinyl, Chinazolinyl, Carbazolyl, Phenazinyl, Phenothiazinyl oder Oxadiazolyl enthält, wobei die Bindung an die Verbindungen der allgemeinen Struktur I über jedes beliebige und mögliche Ringglied des Heteroaryl-Restes erfolgen kann.

Im Zusammenhang mit "Alkyl" versteht man unter dem Begriff "substituiert" im Sinne dieser Erfindung die Substitution eines oder mehrerer Wasserstoffreste durch F, Cl, Br, I, -CN, NH₂, NH-Alkyl, NH-Aryl, NH-Heteroaryl, NH-Cycloalkyl, NH-Alkyl-Aryl, NH-Alkyl-Heteroaryl, NH-Alkyl-OH, N(Alkyl)₂, N(Alkyl-Aryl)₂, N(Alkyl-Heteroaryl)₂, N(Cycloalkyl)₂, N(Alkyl-OH)₂, NO₂, SH, S-Alkyl, S-Aryl, S-Heteroaryl, S-Alkyl-Aryl, S-Alkyl-Heteroaryl, S-Cycloalkyl, S-Alkyl-OH; S-Alkyl-SH, OH, O-Alkyl, O-Aryl, O-Heteroaryl, O-Alkyl-Aryl, O-Alkyl-Heteroaryl, O-Cycloalkyl, O-Alkyl-OH, CHO, C(=O)C₁₋₆-Alkyl, C(=S)C₁₋₆-Alkyl, C(=O)Aryl, C(=S)Aryl, C(=O)C₁₋₆-Alkyl-Aryl, C(=S)C₁₋₆-Alkyl-Aryl, C(=O)-Heteroaryl, C(=S)-Heteroaryl, C(=O)-Cycloalkyl, C(=S)-Cycloalkyl, CO₂H, CO₂-Alkyl, CO₂-Alkyl-Aryl, C(=O)NH₂, C(=O)NH-Alkyl, C(=O)NHAryl, C(=O)NH-Cycloalkyl, C(=O)N(Alkyl)₂, C(=O)N(Alkyl-Aryl)₂, C(=O)N(Alkyl-Heteroaryl)₂, C(=O)N(Cycloalkyl)₂, SO-Alkyl, SO₂-Alkyl, SO₂NH₂, SO₃H, PO(O-C₁₋₆-Alkyl)₂, Si(C₁₋₆-Alkyl)₃, Si(C₃₋₈-Cycloalkyl)₃, Si(CH₂-C₃₋₈-Cycloalkyl)₃, Si(Phenyl)₃, Cycloalkyl, Aryl oder Heteroaryl, wobei unter mehrfach substituierten Resten solche Reste zu verstehen sind, die entweder an verschiedenen oder an gleichen Atomen mehrfach, z. B. zwei- oder dreifach, substituiert sind, beispielsweise dreifach am gleichen C-Atom wie im Falle von CF₃ oder -CH₂CF₃ oder an verschiedenen Stellen wie im Falle von -CH(OH)-CH=CH-CHCl₂. Die Mehrfachsubstitution kann mit dem gleichen oder mit verschiedenen Substituenten erfolgen. Ggf. kann ein Substituent auch seinerseits substituiert sein; so umfaßt -OAlkyl u.a. auch -O-CH₂-CH₂-O-CH₂-CH₂-OH.

In Bezug auf "Aryl", "Heteroaryl" sowie "Cycloalkyl" versteht man im Sinne dieser Erfindung unter "ein- oder mehrfach substituiert" die ein- oder mehrfache, z.B. zwei-, drei- vier- oder fünffache, Substitution eines oder mehrerer Wasserstoffatome des Ringsystems durch F, Cl, Br, I, CN, NH₂, NH-Alkyl, NH-Aryl, NH-Heteroaryl, NH-Alkyl-Aryl, NH-Alkyl-Heteroaryl, NH-Cycloalkyl, NH-Alkyl-OH, N(Alkyl)₂, N(Alkyl-Aryl)₂, N(Alkyl-Heteroaryl)₂, N(Cycloalkyl)₂, N(Alkyl-OH)₂, NO₂, SH, S-Alkyl, S-Cycloalkyl, S-Aryl, S-Heteroaryl, S-Alkyl-Aryl, S-Alkyl-Heteroaryl, S-Cycloalkyl, S-Alkyl-OH, S-Alkyl-SH, OH, O-Alkyl, O-Cycloalkyl, O-Aryl, O-Heteroaryl, O-Alkyl-Aryl, O-Alkyl-Heteroaryl, O-Cycloalkyl, O-Alkyl-OH, CHO, C(=O)C₁₋₆-Alkyl, C(=S)C₁₋₆-Alkyl, C(=O)Aryl, C(=S)Aryl, C(=O)-C₁₋₆-Alkyl-Aryl, C(=S)C₁₋₆-Alkyl-Aryl, C(=O)-Heteroaryl, C(=S)-Heteroaryl, C(=O)-Cycloalkyl, C(=S)-Cycloalkyl, CO₂H, CO₂-Alkyl, CO₂-Alkyl-Aryl, C(=O)NH₂, C(=O)NH-Alkyl, C(=O)NHAryl, C(=O)NH-Cycloalkyl, C(=O)N(Alkyl)₂, C(=O)N(Alkyl-Aryl)₂, C(=O)N(Alkyl-Heteroaryl)₂, C(=O)N(Cycloalkyl)₂, S(O)-Alkyl, S(O)-Aryl, SO₂-Alkyl, SO₂-Aryl, SO₂NH₂, SO₃H, CF₃, =O, =S; Alkyl, Cycloalkyl, Aryl und/oder Heteroaryl; an einem oder ggf. verschiedenen Atomen (wobei ein Substituent ggf. seinerseits substituiert sein kann). Die Mehrfachsubstitution erfolgt dabei mit dem gleichen oder mit unterschiedlichen Substituenten.

Unter dem Begriff Salz ist jegliche Form des erfindungsgemäßen Wirkstoffes zu verstehen, in dem dieser eine ionische Form annimmt bzw. geladen ist und mit einem Gegenion (einem Kation oder Anion) gekoppelt ist bzw. sich in Lösung befindet. Darunter sind auch Komplexe des Wirkstoffes mit anderen Molekülen und Ionen zu verstehen, insbesondere Komplexe, die über ionische Wechselwirkungen komplexiert sind. Insbesondere versteht man darunter (und dies ist auch eine bevorzugte Ausführungsform dieser Erfindung) physiologisch verträgliche Salze, insbesondere physiologisch verträgliche Salze mit Kationen oder Basen und physiologisch verträgliche Salze mit Anionen oder Säuren oder auch ein mit einer physiologisch verträglichen Säure oder einem physiologisch verträglichen Kation gebildetes Salz.

Unter dem Begriff des physiologisch verträglichen Salzes mit Anionen oder Säuren versteht man im Sinne dieser Erfindung Salze mindestens einer der erfindungsgemäßen Verbindungen - meist, beispielsweise am Stickstoff, protoniert - als Kation mit mindestens einem Anion, die physiologisch - insbesondere bei Anwendung im Menschen und/oder Säugetier-verträglich sind. Insbesondere versteht man darunter im Sinne dieser Erfindung das mit einer physiologisch verträglichen Säure gebildete Salz, nämlich Salze des jeweiligen Wirkstoffes mit anorganischen bzw. organischen Säuren, die physiologisch - insbesondere bei Anwendung im Menschen und/oder Säugetier - verträglich sind. Beispiele für physiologisch verträgliche Salze bestimmter Säuren sind Salze der: Salzsäure, Bromwasserstoffsäure, Schwefelsäure, Methansulfonsäure, Ameisensäure, Essigsäure, Oxalsäure, Bernsteinsäure, Apfelsäure, Weinsäure, Mandelsäure, Fumarsäure, Milchsäure, Zitronensäure, Glutaminsäure, Saccharinsäure, Monomethylsebacinsäure, 5-Oxo-prolin, Hexan-1-sulfonsäure, Nicotinsäure, 2-, 3-oder 4-Aminobenzoesäure, 2,4,6-Trimethylbenzoesäure, α-Liponsäure, Acetylglycin, Phosphorsäure, Maleinsäure, Malonsäure, Hippursäure und/oder Asparaginsäure. Besonders bevorzugt ist das Hydrochlorid-Salz, das Citrat und das Hemicitrat.

Unter dem Begriff des mit einer physiologisch verträglichen Säure gebildeten Salzes versteht man im Sinne dieser Erfindung Salze des jeweiligen Wirkstoffes mit anorganischen bzw. organischen Säuren, die physiologisch - insbesondere bei Anwendung im Menschen und/oder Säugetier - verträglich sind. Besonders bevorzugt ist das Hydrochlorid und das Citrat. Beispiele für physiologisch verträgliche Säuren sind: Salzsäure, Bromwasserstoffsäure, Schwefelsäure, Methansulfonsäure, Ameisensäure, Essigsäure, Oxalsäure, Bernsteinsäure, Weinsäure, Mandelsäure, Fumarsäure, Milchsäure, Zitronensäure, Glutaminsäure, Saccharinsäure, Monomethylsebacinsäure, 5-Oxo-prolin, Hexan-1-sulfonsäure, Nicotinsäure, 2-, 3-oder 4-Aminobenzoesäure, 2,4,6-Trimethylbenzoesäure, α-Liponsäure, Acetylglycin, Acetylsalicylsäure, Hippursäure und/oder Asparaginsäure.

Unter dem Begriff des physiologisch verträglichen Salzes mit Kationen oder Basen versteht man im Sinne dieser Erfindung Salze mindestens einer der erfindungsgemäßen Verbindungen - meist einer (deprotonierten) Säure - als Anion mit mindestens einem, vorzugsweise anorganischen, Kation, die physiologisch - insbesondere bei Anwendung im Menschen und/oder Säugetier - verträglich sind. Besonders bevorzugt sind die Salze der Alkali- und Erdalkalimetalle aber auch Ammoniumsalze, insbesondere aber (Mono-) oder (Di-) Natrium-, (Mono-) oder (Di-) Kalium-, Magnesium- oder Calzium-Salze.

Unter dem Begriff des mit einem physiologisch verträglichen Kation gebildeten Salzes versteht man im Sinne dieser Erfindung Salze mindestens einer der jeweiligen Verbindungen als Anion mit mindestens einem anorganischen Kation, das physiologisch - insbesondere bei Anwendung im Menschen und/oder Säugetier - veträglich ist. Besonders bevorzugt sind die Salze der Alkali- und Erdalkalimetalle aber auch Ammoniumsalze, insbesondere aber (Mono-) oder (Di-) Natrium-, (Mono-) oder (Di-) Kalium-, Magnesium- oder Calzium-Salze.

Für eine bevorzugte Ausführungsform der erfindungsgemäßen spirocyclischen Cyclohexan-Derivate gilt, dass
R¹ und R² unabhängig voneinander für H; C₁₋₈-Alkyl, gesättigt oder ungesättigt, verzweigt oder unverzweigt, einfach oder mehrfach substituiert oder unsubstituiert, stehen;
oder die Reste R¹ und R² zusammen einen Ring bilden und CH₂CH₂OCH₂CH₂, CH₂CH₂NR¹¹ CH₂CH₂ oder (CH₂)₃₋₆ bedeuten,
wobei R¹¹ H; C₁₋₈-Alkyl, gesättigt oder ungesättigt, verzweigt oder unverzweigt, einfach oder mehrfach substituiert oder unsubstituiert, bedeutet.

Besonders bevorzugt sind spirocyclische Cyclohexan-Derivate, worin R¹ und R² unabhängig voneinander für CH₃ oder H stehen, wobei R¹ und R² nicht gleichzeitig H bedeuten.

Weiterhin bevorzugt sind spirocyclische Cyclohexan-Derivate, worin
R³ für Cyclopentyl, Cyclohexyl, Phenyl, Benzyl, Naphthyl, Anthracenyl, Thiophenyl, Benzothiophenyl, Furyl, Benzofuranyl, Benzodioxolanyl, Indolyl, Indanyl, Benzodioxanyl, Pyrrolyl, Pyridyl, Pyrimidyl oder Pyrazinyl, jeweils unsubstituiert oder einfach oder mehrfach substituiert; über eine gesättigte, unverzweigte C₁₋₂-Alkyl-Gruppe gebundenen C₅₋₆-Cycloalkyl, Phenyl, Naphthyl, Anthracenyl, Thiophenyl, Benzothiophenyl, Pyridyl, Furyl, Benzofuranyl, Benzodioxolanyl, Indolyl, Indanyl, Benzodioxanyl, Pyrrolyl, Pyrimidyl oder Pyrazinyl, jeweils unsubstituiert oder einfach oder mehrfach substituiert, steht;
   insbesondere
R³ Phenyl, Furyl, Thiophenyl, Naphthyl, Benzyl, Benzofuranyl, Indolyl, Indanyl, Benzodioxanyl, Benzodioxolanyl, Pyridyl, Pyrimidyl, Pyrazinyl oder Benzothiophenyl, jeweils unsubstituiert oder einfach oder mehrfach substituiert; über eine gesättigte, unverzweigte C₁₋₂-Alkyl-Gruppe gebundenen Phenyl, Furyl oder Thiophenyl, jeweils unsubstituiert oder einfach oder mehrfach substituiert, bedeutet.

Besonders bevorzugt sind spirocyclische Cyclohexan-Derivate, worin R³ für Phenyl, Phenethyl, Thiophenyl, Pyridyl oder Benzyl, jeweils substituiert oder unsubstituiert, steht, besonders bevorzugt für Phenyl.

Ebenfalls besonders bevorzugt sind spirocyclische Cyclohexan-Derivate, worin R³ Phenyl bedeutet, unsubstituiert oder mit F, Cl, CN, OCH₃, OCH₂CH₃, CH₃, CF₃ oder OH einfach oder mehrfach substituiert, insbesondere 3-Fluorphenyl und 4-Fluorphenyl.

Außerdem bevorzugt sind spirocyclische Cyclohexan-Derivate, worin R⁵ für H, C₁₋₅-Alkyl, verzweigt oder unverzweigt, unsubstituiert oder einfach oder mehrfach substituiert oder COOR¹³ steht und R⁶ H oder C₁₋₅-Alkyl bedeutet.

Darüber hinaus sind auch spirocyclische Cyclohexan-Derivate bevorzugt, worin R⁷, R⁸, R⁹ und R¹⁰ unabhängig voneinander für H; C₁₋₅-Alkyl, verzweigt oder unverzweigt, unsubstituiert oder ein- oder mehrfach substituiert; F, Cl, Br, I, CF₃, OH, OCH₃, NH₂, COOH, COOCH₃, NHCH₃ oder N(CH₃)₂ oder NO₂ stehen.

Besonders bevorzugt sind spirocyclische Cyclohexan-Derivate, worin die Reste R⁵-R¹⁰ H bedeuten.

Ganz besonders bevorzugt sind spirocyclische Cyclohexan-Derivate aus der Gruppe
*N,N*-Dimethyl-*N*-{4-phenyl-1',3',4',5'-tetrahydro-spiro[cyclohexan-1,1'-pyrano[4,3-b]indol]-4-yl}amin
*N,N*-Dimethyl-*N*-{4-phenyl-1',3',4',5'-tetrahydro-spiro[cyclohexan-1,1'-pyrano[4,3-b]indol]-4-yl}amin-triflat
*N,N*-Dimethyl-*N*-{4-phenyl-1',3',4',5'-tetrahydro-spiro[cyclohexan-1,1'-pyrano[4,3-b]indol]-4-yl}amin-citrat
*N,N*-Dimethyl-*N*-{4-(4-fluorphenyl)-1',3',4',5'-tetrahydro-spiro[cyclohexan-1,1'-pyrano[4,3-b]indol]-4-yl}ammonium-triflat
*N,N*-Dimethyl-*N*-{4-(3-fluorphenyl)-1',3',4',5'-tetrahydro-spiro[cyclohexan-1,1'-pyrano[4,3-b]indol]-4-yl}ammonium-triflat
*N,N*-Dimethyl-*N*-{4-phenyl-spiro[cyclohexan-1,4'-1',4'-dihydro-2'*H*-3'-oxa-9'-thiafluoren]-4-yl}amin
*N,N*-Dimethyl-*N*-{4-phenyl-spiro[cyclohexan-1,4'-1',4'-dihydro-2'*H*-3'-oxa-9'-thiafluoren]-4-yl}amin-triflat
*N,N*-Dimethyl-*N*-{4-(4-fluorphenyl)-spiro[cyclohexan-1,4'-1',4'-dihydro-2'*H*-3'-oxa-9'-thiafluoren]-4-yl}ammonium-triflat
*N,N*-Dimethyl-*N*-{4-(3-fluorphenyl)-spiro[cyclohexan-1,4'-1',4'-dihydro-2'*H*-3'-oxa-9'-thiafluoren]-4-yl}ammonium-triflat
*N,N*-Dimethyl-*N*-{4-phenyl-1',4'-dihydrospiro[cyclohexan-1,4'-2'*H*-3',9'-dithiafluoren]-4-yl}ammonium-methansulfonat
*N,N*-Dimethyl-*N*-{4-phenyl-1',4'-dihydrospiro[cyclohexan-1,4'-2'*H*-9'-oxa-3'-thiafluoren]-4-yl}ammonium-methansulfonat
*N,N*-Dimethyl-*N*-{4-pyridin-1',3',4',5'-tetrahydro-spiro[cyclohexan-1,1'-pyrano[4,3-b]indol]-4-yl}amin
*N,N*-Dimethyl-*N*-{4-pyridin-1',3',4',5'-tetrahydro-spiro[cyclohexan-1,1'-pyrano[4,3-b]indol]-4-yl}amin-citrat
*N,N*-Dimethyl-*N*-{4-phenyl-1',4'-dihydrospiro[cyclohexan-1,4'-2'*H*-3',9'-dioxafluoren]-4-yl}ammonium-methansulfonat
in Form des Razemats; der Enantiomere, Diastereomere, Mischungen der Enantiomere oder Diastereomere oder eines einzelnen Enantiomers oder Diastereomers; der Basen und/oder Salze physiologisch verträglicher Säuren oder Kationen.

Die erfindungsgemäßen Substanzen wirken beispielsweise auf den im Zusammenhang mit verschiedenen Erkrankungen relevanten µ-Opioid-Rezeptor, sodass sie sich als pharmazeutischer Wirkstoff in einem Arzneimittel eignen. Ein weiterer Gegenstand der Erfindung sind daher Arzneimittel enthaltend wenigstens ein erfindungsgemäßes spirocyclisches Cyclohexan-Derivat, sowie gegebenenfalls geeignete Zusatz- und/oder Hilfsstoffe und/oder gegebenenfalls weitere Wirkstoffe.

Die erfindungsgemäßen Arzneimittel enthalten neben mindestens einem erfindungsgemäßen spirocyclischen Cyclohexan-Derivat gegebenenfalls geeignete Zusatz- und/oder Hilfsstoffe, so auch Trägermaterialien, Füllstoffe, Lösungsmittel, Verdünnungsmittel, Farbstoffe und/oder Bindemittel und können als flüssige Arzneiformen in Form von Injektionslösungen, Tropfen oder Säfte, als halbfeste Arzneiformen in Form von Granulaten, Tabletten, Pellets, Patches, Kapseln, Pflaster/Sprühpflaster oder Aerosolen verabreicht werden. Die Auswahl der Hilfsstoffe etc. sowie die einzusetzenden Mengen derselben hängen davon ab, ob das Arzneimittel oral, peroral, parenteral, intravenös, intraperitoneal, intradermal, intramuskulär, intranasal, buccal, rektal oder örtlich, zum Beispiel auf die Haut, die Schleimhäute oder in die Augen, appliziert werden soll. Für die orale Applikation eignen sich Zubereitungen in Form von Tabletten, Dragees, Kapseln, Granulaten, Tropfen, Säften und Sirupen, für die parenterale, topische und inhalative Applikation Lösungen, Suspensionen, leicht rekonstituierbare Trockenzubereitungen sowie Sprays. Erfindungsgemäße spirocyclische Cyclohexan-Derivate in einem Depot, in gelöster Form oder in einem Pflaster, gegebenenfalls unter Zusatz von die Hautpenetration fördernden Mitteln, sind geeignete perkutane Applikationszubereitungen. Oral oder perkutan anwendbare Zubereitungsformen können die erfindungsgemäßen spirocyclischen Cyclohexan-Derivate verzögert freisetzen. Die erfindungsgemäßen spirocyclischen Cyclohexan-Derivate können auch in parenteralen Langzeitdepotformen wie z. B. Implantaten oder implantierten Pumpen angewendet werden. Prinzipiell können den erfindungsgemäßen Arzneimitteln andere dem Fachmann bekannte weitere Wirkstoffe zugesetzt werden.

Die an den Patienten zu verabreichende Wirkstoffmenge variiert in Abhängigkeit vom Gewicht des Patienten, von der Applikationsart, der Indikation und dem Schweregrad der Erkrankung. Üblicherweise werden 0,00005 bis 50 mg/kg, bevorzugt 0,01 bis 5 mg/kg wenigstens eines erfindungsgemäßen spirocyclischen Cyclohexan-Derivats appliziert.

Für alle vorstehenden Formen der erfindungsgemäßen Arzneimittel ist es besonders bevorzugt, wenn das Arzneimittel neben wenigstens einem spirocyclischen Cyclohexan-Derivat noch einen weiteren Wirkstoff, insbesondere ein Opioid, vorzugsweise ein starkes Opioid, insbesondere Morphin, oder ein Anesthetikum, vorzugsweise Hexobarbital oder Halothan, enthält.

In einer bevorzugten Form des Arzneimittel liegt ein enthaltenes erfindungsgemäßes spirocyclisches Cyclohexan-Derivat als reines Diastereomer und/oder Enantiomer, als Razemat oder als nicht-äquimolare oder äquimolare Mischung der Diastereomere und/oder Enantiomere vor.

Der µ-Opioid-Rezeptor, aber auch die anderen Opioid-Rezeptoren, wurden insbesondere im Schmerzgeschehen identifiziert. Entsprechend können erfindungsgemäße spirocyclische Cyclohexan-Derivate zur Herstellung eines Arzneimittels zur Behandlung von Schmerz, insbesondere von akutem, neuropathischem oder chronischem Schmerz, verwendet werden.

Ein weiterer Gegenstand der Erfindung ist daher die Verwendung eines erfindungsgemäßen spirocyclischen Cyclohexan-Derivats zur Herstellung eines Arzneimittels zur Behandlung von Schmerz, insbesondere von akutem, viszeralem, neuropathischem oder chronischem Schmerz.

Ein weiterer Gegenstand der Erfindung ist die Verwendung eines erfindungsgemäßen spirocyclischen Cyclohexan-Derivats zur Herstellung eines Arzneimittels zur Behandlung von Angstzuständen, von Stress und mit Stress verbundenen Syndromen, Depressionen, Epilepsie, Alzheimer Erkrankung, seniler Demenz, allgemeinen kognitiven Dysfunktionen, Lern- und Gedächtnis-Störungen (als Nootropikum), Entzugserscheinungen, Alkohol- und/oder Drogen- und/oder Medikamentenmißbrauch und/oder -abhängigkeit, sexuellen Dysfunktionen, cardiovaskulären Erkrankungen, Hypotension, Hypertension, Tinitus, Pruritus, Migräne, Schwerhörigkeit, mangelnder Darmmotilität, gestörter Nahrungsaufnahme, Anorexie, Fettsucht, lokomotorischen Störungen, Diarrhoe, Kachexie, Harninkontinenz bzw. als Muskelrelaxanz, Antikonvulsivum oder Anesthetikum bzw. zur Coadministration bei Behandlung mit einem opioiden Analgetikum oder mit einem Anesthetikum, zur Diurese oder Antinatriurese, Anxiolyse, zur Modulation der Bewegungsaktivität, zur Modulation der Neurotransmitter-Ausschüttung und Behandlung damit verbundener neurodegenerativer Erkrankungen, zur Behandlung von Entzugserscheinungen und/oder zur Reduzierung des Suchtpotentials von Opioiden.

Dabei kann es in einer der vorstehenden Verwendungen bevorzugt sein, wenn ein verwendetes spirocyclisches Cyclohexan-Derivat als reines Diastereomer und/oder Enantiomer, als Razemat oder als nicht-äquimolare oder äquimolare Mischung der Diastereomere und/oder Enantiomere vorliegt.

Ein weiterer Gegenstand der Erfindung ist ein Verfahren zur Behandlung, insbesondere in einer der vorgenannten Indikationen, eines nichthumanen Säugetieres oder Menschen, das oder der eine Behandlung von Schmerzen, insbesondere chronischer Schmerzen, benötigt, durch Verabreichung einer therapeutisch wiksamen Dosis eines erfindungsgemäßen spirocyclischen Cyclohexan-Derivats, oder eines erfindungsgemäßen Arzneimittels.

Ein weiterer Gegenstand der Erfindung ist ein Verfahren zur Herstellung der erfindungsgemäßen spirocyclischen Cyclohexan-Derivate wie in der folgenden Beschreibung und Beispielen ausgeführt. Insbesondere geeignet ist dabei ein, im folgenden Hauptverfahren genanntes, Verfahren zur Herstellung eines erfindungsgemäßen spirocyclischen Cyclohexan-Derivats mit folgenden Schritten,
wobei X, W, R¹, R², R³, R⁵, R⁶, R⁷, R⁸, R⁹ und R¹⁰ die für erfindungsgemäße Verbindungen gemäß Formel I angegebene Bedeutung haben,
und
R⁰¹ und R⁰² die für erfindungsgemäße Verbindungen gemäß Formel 1 für R¹ und R² angegebene Bedeutung haben und zusätzlich unabhängig voneinander für eine Schutzgruppe stehen können:

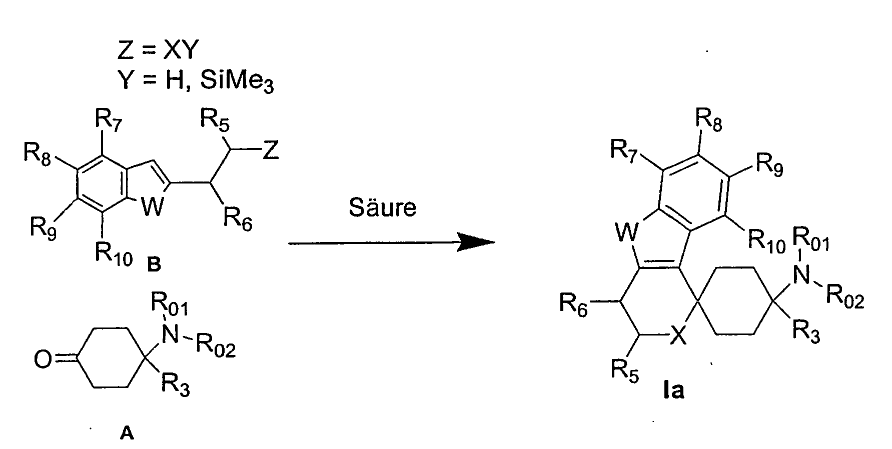

Zur Herstellung der Verbindungen der allgemeinen Formel la werden Ketone der allgemeinen Formel A mit Heteroaromaten der allgemeinen Formel B unter Zugabe von Säure oder deren Trimethylsilylester, beispielsweise Trifluormethansulfonsäuretrimethylsilylester, Essigsäure, Phosphorsäure, Methansulfonsäure oder Trifluoressigsäure in einem geeigneten Lösungsmittel, beispielsweise Dichlorethan, Dichlormethan, Chloroform, Acetonitril, Diethylether oder Nitromethan, umgesetzt.

Alternativ kann die Herstellung auch nach folgendem Schema erfolgen, wobei X, W, R³, R⁵, R⁶, R⁷, R⁸, R⁹ und R¹⁰ die für erfindungsgemäße Verbindungen gemäß Formel I angegebene Bedeutung haben,
und
R⁰¹ und R⁰² die für erfindungsgemäße Verbindungen gemäß Formel I für R¹ und R² angegebene Bedeutung haben und zusätzlich unabhängig voneinander für eine Schutzgruppe stehen können.

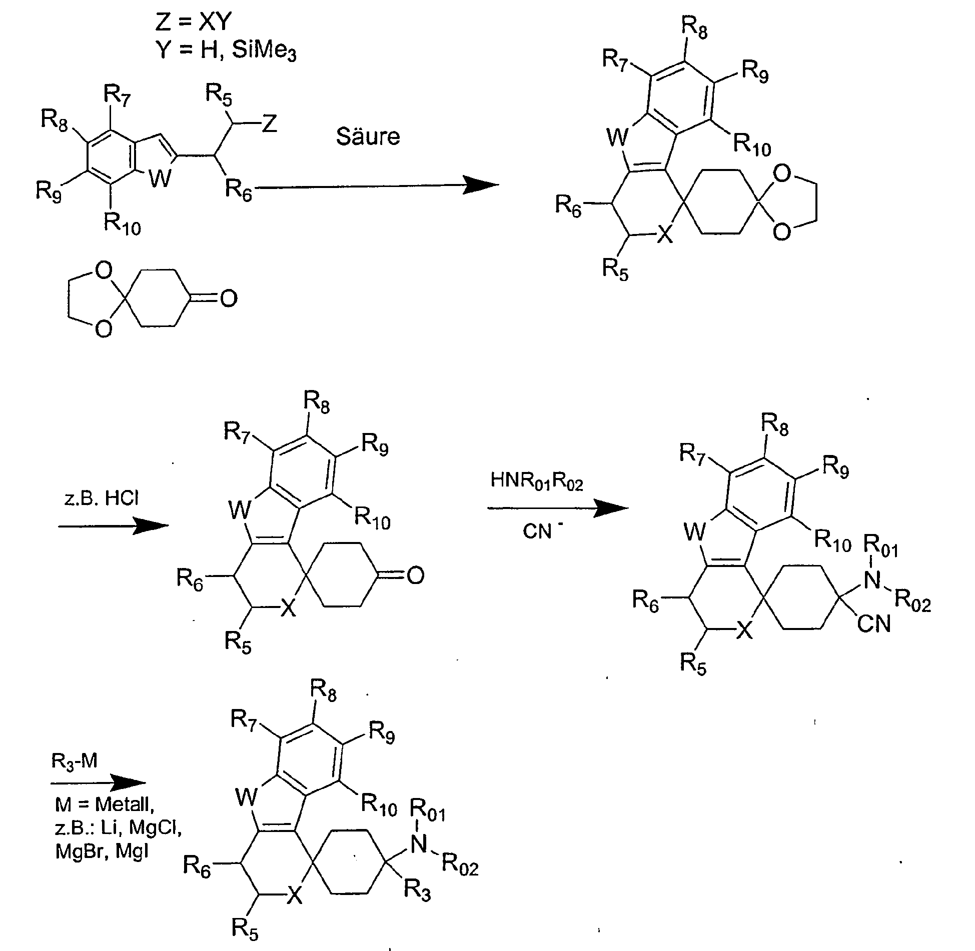

Spirocyclische Cyclohexanderivate der allgemeinen Formel I, bei denen X NR¹⁷ und R¹⁷ COR¹² oder SO₂R¹² bedeutet, können durch die Umsetzung von spirocyclischen Cyclohexanderivaten der allgemeinen Formel I, bei denen X NH bedeutet, durch Umsetzung mit einem Anhydrid oder einem Säurechlorid unter Zugabe einer Base, beispielsweise Triethylamin, erhalten werden. Vorzugsweise findet diese Reaktion unter Mikrowelleneinstrahlung statt.

Spirocyclische Cyclohexanderivate der allgemeinen Formel I, bei denen X SO oder SO₂ bedeuten, können durch Umsetzung von spirocyclischen Cyclohexanderivaten der allgemeinen Formel I, bei denen X S bedeutet, mit einem Oxidationsmittel, beispielsweise H₂O₂, erhalten werden.

### Beispiele

Die folgenden Beispiele dienen zur näheren Erläuterung der Erfindung, schränken aber den allgemeinen Erfindungsgedanken nicht ein.

Die Ausbeuten der hergestellten Verbindungen sind nicht optimiert.

Alle Temperaturen sind unkorrigiert.

Die Angabe "Ether" bedeutet Diethylether, "EE" Ethylacetat und "DCM" Dichlormethan. Die Angabe "Äquivalente" bedeutet Stoffmengenäquivalente, "Smp." Schmelzpunkt bzw. Schmelzbereich, "Zers." Zersetzung, "RT" Raumtemperatur , "abs." absolut (wasserfrei), ,"rac." racemisch , "konz." konzentriert, "min" Minuten, "h" Stunden, "d" Tage, "Vol.%" Volumenprozent, "m%" Massenprozent und "M" ist eine Konzentrationsangabe in mol/I.

Als stationäre Phase für die Säulenchromatographie wurde Kieselgel 60 (0.040 - 0.063 mm) der Firma E. Merck, Darmstadt, eingesetzt.

Die dünnschicht-chromatographischen Untersuchungen wurden mit HPTLC-Fertigplatten, Kieselgel 60 F 254, der Firma E. Merck, Darmstadt, durchgeführt.

Die Mischungsverhältnisse von Laufmitteln für chromatographische Untersuchungen sind stets in Volumen/Volumen angegeben.

Die im folgenden eingesetzten Verbindungen waren entweder kommerziell erhältlich, oder ihre Herstellung ist aus dem Stand der Technik bekannt oder in für den Fachmann offensichtlicher Weise aus dem Stand der Technik abgeleitet worden.

### Beispiel 1:

### N,N-Dimethyl-N-{4-phenyl-1',3',4',5'-tetrahydro-spiro[cyclohexan-1,1'-pyrano[4,3-b]indol]-4-yl}amin

### Beispiel 2:

### N,N-Dimethyl-N-{4-phenyl-1',3',4',5'-tetrahydro-spiro[cyclohexan-1,1'-pyrano[4,3-b]indol]-4-yl}amin-triflat

### Beispiel 3

### N,N-Dimethyl-N-{4-phenyl-1',3',4',5'-tetrahydro-spiro[cyclohexan-1,1'-pyrano[4,3-b]indol]-4-yl}amin-citrat

### Methode A

Unter Argon wurden 4-Dimethylamino-4-phenyl-cyclohexanon (217 mg, 1 mmol) und 2-(1*H-*Indol-2-yl)ethanol (161 mg, 1 mmol) in Eisessig (4 ml) und konz. Phosporsäure (1 ml) gelöst. Der Ansatz wurde 20 h bei RT gerührt. Zur Aufarbeitung wurde der ausgefallene Feststoff abgesaugt, in 1 N NaOH (30 ml) suspendiert und 1 h bei Raumtemperatur gerührt. Der Spiroether (Beispiel 1) wurde anschließend in einer Ausbeute von 61 % (200 mg) mit einem Schmelzpunkt von 311-314 °C als beigefarbener Feststoff abgesaugt.

### Methode B

Unter Argon wurde 4-Dimethylamino-4-phenyl-cyclohexanon (217 mg, 1 mmol) zusammen mit 2-(1*H-*Indol-2-yl)-ethanol (161 mg, 1 mmol) in absolutem Dichlormethan (10 ml) vorgelegt. Anschließend erfolgte die Zugabe von Trifluormethansulfonsäuretrimethylsilylester (0,2 ml, 1,03 mmol). Der Ansatz wurde 19 h bei RT gerührt. Zur Aufarbeitung wurde das ausgefallene Triflat abgesaugt und mit Dichlormethan (2 × 10 ml) gewaschen. Beispiel 2 wurde so in einer Ausbeute von 99 % (504 mg) als farbloser Feststoff mit einem Schmelzpunkt von 249-253 °C erhalten.

### Methode C

Der nach Methode A gewonnene Spiroether (Beispiel 1) (220 mg, 0,61 mmol) wurde in Methanol (100 ml) suspendiert und mit Trifluormethansulfonsäure (0,11 ml, 1,2 mmol) versetzt. Die Reaktionsmischung wurde 15 min unter Rückfluß erhitzt, wobei eine klare Lösung entstand. Das Reaktionsvolumen wurde auf 10 ml reduziert. Nach Abkühlen wurde das Triflat (Beispiel 2) in einer Ausbeute von 70 % (217 mg) abgesaugt.

### Herstellung des Citrats

Das Triflat (Beispiel 2) wurde in 1 N Natronlauge suspendiert (15 ml) und 15 min gerührt. Der dabei erhaltene Feststoff wurde abgesaugt, mit Wasser (2 × 10 ml) gewaschen und getrocknet. Die freie Base (Beispiel 1) wurde dabei in einer Ausbeute von 66 % als beigefarbener Feststoff in einer Ausbeute von 66 % (239 mg) gewonnen. Zur Herstellung des Citrates wurde die Base (229 mg, 0,63 mmol) in Methanol (90 ml) bei 50 °C suspendiert und mit Citronensäure (122 mg, 0,63 mmol), gelöst in warmen Methanol (10 ml), versetzt. Die Reaktionsmischung wurde 2 h bei RT gerührt und der ausgefallene Feststoff abgesaugt. Das Citrat (Beispiel 3) wurde dabei in einer Ausbeute von 62 % (216 mg) als weißer Feststoff mit einem Schmelzpunkt von 249-243 °C erhalten.

### Beispiel 4:

### N,N-Dimethyl-N-{4-(4-fluorphenyl)-1',3',4',5'-tetrahydro-spiro[cyclohexan-1,1'-pyrano[4,3-b]indol]-4-yl}ammonium-triflat

Unter Argon wurde 4-Dimethylamino-4-(4-fluorphenyl)-cyclohexanon (235 mg, 1 mmol) zusammen mit 2-(1*H-*Indol-2-yl)-ethanol (161 mg, 1 mmol) in absolutem Dichlormethan (10 ml) vorgelegt. Anschließend erfolgte die Zugabe von Trifluormethansulfonsäuretrimethylsilylester (0,2 ml, 1,03 mmol). Der Ansatz wurde 20 h bei RT gerührt. Zur Aufarbeitung wurde das ausgefallene Triflat abgesaugt und mit Dichlormethan (2 × 10 ml) gewaschen. Das Triflat (Beispiel 4) wurde so in einer Ausbeute von 94 % (495 mg) als lilafarbener Feststoff mit einem Schmelzpunkt von 232-233 °C erhalten.

### Beispiel 5:

### N,N-Dimethyl-N-{4-(3-fluorphenyl)-1',3',4',5'-tetrahydro-spiro[cyclohexan-1,1'-pyrano[4,3-b]indol]-4-yl}ammonium-triflat

Unter Argon wurde 4-Dimethylamino-4-(3-fluorphenyl)-cyclohexanon (235 mg, 1 mmol) zusammen mit 2-(1*H-*Indol-2-yl)-ethanol (161 mg, 1 mmol) in absolutem Dichlormethan (10 ml) vorgelegt. Anschließend erfolgte die Zugabe von Trifluormethansulfonsäuretrimethylsilylester (0,2 ml, 1,03 mmol). Der Ansatz wurde 20 h bei RT gerührt. Zur Aufarbeitung wurde das ausgefallene Triflat abgesaugt und mit Dichlormethan (2 × 10 ml) gewaschen. Das Triflat (Beispiel 5) wurde in einer Ausbeute von 98 % (518 mg) als lilafarbener Feststoff mit einem Schmelzpunkt von 174-189 °C erhalten.

### Beispiel 6:

### N,N-Dimethyl-N-{4-phenyl-spiro[cyclohexan-1,4'-1',4'-dihydro-2'H-3'-oxa-9'-thiafluoren]-4-yl}amin

### Beispiel 7:

### N,N-Dimethyl-N-{4-phenyl-spiro[cyclohexan-1,4'-1',4'-dihydro-2'H-3'-oxa-9'-thiafluoren]-4-yl}amin-triflat

### Methode A

Unter Argon wurden 4-Dimethylamino-4-phenyl-cyclohexanon (217 mg, 1 mmol) und 2-Benzo[b]thiophen-2-ylethanol (178 mg, 1 mmol) in Eisessig (4 ml) und konz. Phosporsäure (1 ml) gelöst. Der Ansatz wurde 3 d bei RT gerührt. Zur Aufarbeitung wurde das Reaktionsgemisch mit Eis (30 g) versetzt und mit NaHCO₃ (9 g, 0,107 mol) neutralisiert. Nach der Zugabe von Diethylether (70 ml) wurde 30 min gerührt. Die organische Phase wurde abgetrennt und die verbleibende wäßrige Phase mit Diethylether (2 × 50 ml) extrahiert. Die vereinigten organischen Extrakte wurden über Na₂SO₄ getrocknet und eingeengt. Der Rückstand (386 mg) wurde chromatographisch an Kieselgel (35 g) mit Ethylacetat/Methanol (4:1) getrennt und der Spiroether (Beispiel 6) dabei in einer Ausbeute von 14 % (53 mg) als farbloser Feststoff mit einem Schmelzpunkt von 262-263 °C isoliert.

### Methode B

Unter Argon wurde 4-Dimethylamino-4-phenyl-cyclohexanon (217 mg, 1 mmol) zusammen mit 2-Benzo[b]thiophen-2-ylethanol (178 mg, 1 mmol) in absolutem Dichlormethan (10 ml) vorgelegt. Anschließend erfolgte die Zugabe von Trifluoressigsäure (3 ml). Der Ansatz wurde 24 h bei RT gerührt. Zur Aufarbeitung wurde das Reaktionsgemisch mit Eis (30 g) versetzt und mit NaHCO₃ (3,4 g, 0,04 mol) neutralisiert. Danach wurde 5N NaOH (0,5 ml) hinzugefügt. Die wäßrige Phase wurde abgetrennt und mit Dichlormethan (2 × 30 ml) extrahiert. Die vereinigten organischen Extrakte wurden über Na₂SO₄ getrocknet und eingeengt. Der Rückstand (400 mg) wurde chromatographisch an Kieselgel (40 g) mit Ethylacetat/Methanol (4 : 1) und Methanol getrennt und der Spiroether (Beispiel 6) in einer Ausbeute von 76 % (286 mg) als farbloser Feststoff isoliert.

### Methode C

Unter Argon wurde 4-Dimethylamino-4-phenyl-cyclohexanon (217 mg, 1 mmol) zusammen mit 2-Benzo[b]thiophen-2-ylethanol (178 mg, 1 mmol) in absolutem dichlormethan (5 ml) gelöst. Anschließend erfolgte die Zugabe von Methansulfonsäure (3 ml). Der Ansatz wurde 24 h bei RT gerührt. Zur Aufarbeitung wurde das Reaktionsgemisch mit Eis (30 g) versetzt. Dabei fiel ein farbloser Feststoff aus, der in 1 N Natronlauge (10 ml) und Trichlormethan (30 ml) suspendiert und 30 min gerührt wurde. Die organische Phase wurde abgetrennt und die wäßrige Phase mit Trichlormethan (30 ml) extrahiert. Die organischen Extrakte wurden nach dem Trocknen eingeengt und der Rückstand (295 mg) chromatographisch an Kieselgel (40 g) mit Ethylacetat/Methanol (4 : 1) getrennt. Dabei wurde der Spiroether (Beispiel 6) in einer Ausbeute von 35 % (102 mg) als farbloser Feststoff isoliert.

### Methode D

Unter Argon wurde 4-Dimethylamino-4-phenyl-cyclohexanon (217 mg, 1 mmol) zusammen mit 2-Benzo[b]thiophen-2-ylethanol (178 mg, 1 mmol) in absolutem Dichlormethan (10 ml) vorgelegt. Anschließend erfolgte die Zugabe von Trifluormethansulfonsäuretrimethylsilylester (0,2 ml, 1,03 mmol). Der Ansatz wurde 20 h bei RT gerührt. Zur Aufarbeitung wurde das ausgefallene Triflat abgesaugt und mit Dichlormethan (2 × 10 ml) gewaschen. Das Triflat (Beispiel 7) wurde in einer Ausbeute von 99 % (523 mg) als farbloser Feststoff mit einem Schmelzpunkt von 209-211 °C erhalten.

### Beispiel 8:

### N,N-Dimethyl-N-{4-(4-fluorphenyl)-spiro[cyclohexan-1,4'-1',4'-dihydro-2'H-3'-oxa-9'-thiafluoren]-4-yl}ammonium-triflat

Unter Argon wurde 4-Dimethylamino-4-(4-fluorphenyl)-cyclohexanon (235 mg, 1 mmol) zusammen mit 2-Benzo[b]thiophen-2-ylethanol (178 mg, 1 mmol) in absolutem Dichlormethan (10 ml) vorgelegt. Anschließend erfolgte die Zugabe von Trifluormethansulfonsäuretrimethylsilylester (0,2 ml, 1,03 mmol). Der Ansatz wurde 3 d bei RT gerührt. Zur Aufarbeitung wurde das ausgefallene Triflat abgesaugt und mit Dichlormethan (2 × 10 ml) gewaschen Der Spiroether (Beispiel 8) wurde in einer Ausbeute von 97 % (527 mg) als farbloser Feststoff mit einem Schmelzpunkt von 218-221°C erhalten.

### Beispiel 9:

### N,N-Dimethyl-N-{4-(3-fluorphenyl)-spiro[cyclohexan-1,4'-1',4'-dihydro-2'H-3'-oxa-9'-thiafluoren]-4-yl}ammonium-triflat

Unter Argon wurde 4-Dimethylamino-4-(3-fluorphenyl)-cyclohexanon (235 mg, 1 mmol) zusammen mit 2-Benzo[b]thiophen-2-ylethanol (178 mg, 1 mmol) in absolutem Dichlormethan (10 ml) vorgelegt. Anschließend erfolgte die Zugabe von Trifluormethansulfonsäuretrimethylsilylester (0,2 ml, 1,03 mmol). Der Ansatz wurde 21 h bei RT gerührt. Zur Aufarbeitung wurde das ausgefallene Triflat abgesaugt und mit Dichlormethan (2 × 10 ml) gewaschen. Das Triflat (Beispiel 9) wurde in einer Ausbeute von 90 % (492 mg) als farbloser Feststoff mit einem Schmelzpunkt von 227-231 °C erhalten.

### Beispiel 10:

### N,N-Dimethyl-N-{4-phenyl-1',4'-dihydrospiro[cyclohexan-1,4'-2'H-3',9'-dithiafluoren]-4-yl}ammonium-methansulfonat

2-(2-Bromethyl)-benzo[b]thiophen (5,4 g, 21 mmol) wurde mit einem Gemisch aus Natriumthiosulfat (6,64 g, 42 mmol), Ethanol (70 ml) und Wasser (40 ml) versetzt und 3,5 h unter Rückfluß gekocht. Dabei entstand eine klare Lösung. Die Reaktionslösung wurde eingeengt und der Rückstand (farbloser Feststoff), der das entsprechende Bunte-Salz enthielt. Das Rohprodukt des Bunte-Salzes (theoretisch 21 mmol) wurde in 50-proz. Phosphorsäure (130 ml) und Dichlormethan (80 ml) aufgenommen und 80 h bei Raumtemperatur und 8 h bei 40 °C gerührt. Die Reaktionsmischung wurde mit Wasser (170 ml) und Dichlormethan (60 ml) verdünnt und die Phasen getrennt. Die wäßrige Phase wurde mit Dichlormethan (1 × 50 ml) extrahiert und die organischen Phasen vereinigt. Nach dem Waschen der organischen Phase mit Wasser (1 × 50 ml) wurde sie getrocknet und eingeengt. 2-Benzo[b]thiophen-2-yl-ethanthiol wurde dabei als farbloses Öl (2,91 g) in einer Ausbeute von 73 % erhalten.

Unter Argon wurde 4-Dimethylamino-4-phenyl-cyclohexanon (217 mg, 1 mmol) zusammen mit 2-(Benzo[b]thiophen-2-yl)ethanthiol (194 mg, 1 mmol) in absolutem Diethylether (8 ml) vorgelegt. Anschließend erfolgte die Zugabe von Methansulfonsäure (2,8 ml). Es begann sofort ein Reaktionsprodukt auszufallen. Der Ansatz wurde 4,5 h bei RT gerührt. Zur Aufarbeitung wurde der ausgefallene farblose Feststoff abgesaugt und mit Diethylether gewaschen (2 × 10 ml). Das Methansulfonat (Beispiel 10) wurde dabei in einer Ausbeute von 99 % (484 mg) und einem Schmelzpunkt von 178-182 °C gewonnen.

### Beispiel 11

### N,N-Dimethyl-N-{4-phenyl-1',4'-dihydrospiro[cyclohexan-1,4'-2'H-9'-oxa-3'-thiafluoren]-4-yl}ammonium-methansulfonat

Zu einer Suspension von Triphenylphosphindibromid (7,5 g, 17,9 mmol) in trockenem Acetonitril (20 ml) wurde unter Argon und Wasserkühlung eine Lösung aus Benzo[b]furan-2-ylethanol (2,9 g, 17,9 mmol) in abs. Acetonitril (30 ml) innerhalb von 10 min getropft. Gegen Ende der Zugabe entstand eine klare Lösung. Es wurde 24 h bei RT gerührt, wobei nach und nach ein Feststoff (Ph₃PO) ausfiel. Er wurde abgesaugt und mit Aceton gewaschen. Das Filtrat wurde am Rotationsverdampfer eingeengt. Der teilweise feste Rückstand wurde noch einmal mit Aceton versetzt. Erneut wurde Ph₃PO abgesaugt und das Filtrat wieder eingeengt. Der ölige Rückstand wurde durch Chromatographie an Kieselgel G (80 g; Cyclohexan) gereinigt. 2-(2-Bromethyl)-benzofuran konnte in einer Ausbeute von 81 % (3,25 g) gewonnen werden.

Zu einer Mischung von 2-(2-Bromethyl)-benzofuran (3,22 g, 14,3 mmol) und Ethanol (50 ml) wurde eine Lösung aus Natriumthiosulfat (4,5 g, 28,6 mmol) und Wasser (50 ml) gegeben. Es entstand eine trübe Lösung, die beim Erwärmen klar wurde. Es wurde 4 h zum Rückfluß erhitzt und anschließend 18 h bei RT gerührt. Die klare Lösung wurde eingeengt. Um Verunreinigungen zu entfernen, wurde der feste Rückstand mit Dichlormethan versetzt und 10 min kräftig gerührt. Dann wurde abgesaugt und mit Dichlormethan (2 × 20 ml) gewaschen. Der erhaltene Feststoff wurde in einem 500 ml-Einhalskolben unter Rühren mit 50-proz. Phosphorsäure (85 ml) und anschließend mit Ether (50 ml) versetzt. Nach 24 h Rühren bei RT war ein Dreiphasensystem entstanden, zu dem Ether (100 ml) gegeben wurde. Die Mischung wurde unter Rühren 6 h zum Rückfluß erhitzt, wobei ein Zweiphasensystem entstand. Die Phasen wurden getrennt. Die wäßrige Phase wurde mit Diethylether (2 × 50 ml) extrahiert. Die vereinigten organischen Extrakte wurden mit Wasser gewaschen (2 × 50 ml), über Na₂SO₄ getrocknet und nach dem Trocknen eingeengt. Der Rückstand war ein gelbes Öl mit festen Bestandteilen. Der Feststoff wurde abgesaugt und mit Ether gewaschen (158 mg, mit unbekannter Struktur). Das Filtrat wurde eingeengt. 2-Benzofuran-2-yl-ethanthiol wurde so als hellbraunes Öl in einer Ausbeute von 83 % (2,13 g) erhalten.

4-Dimethylamino-4-phenyl-cyclohexanon (217,3 mg, 1 mmol) und 2-Benzofuran-2-yl-ethanthiol (178,2 mg, 1 mmol) wurden unter Argon in Ether (8 ml) gelöst. Zu der klaren hellbraunen Lösung wurde Methansulfonsäure (2,8 ml) gegeben. Es wurde 18 h bei RT gerührt, dann die klare Reaktionsmischung mit Ether versetzt (40 ml), bis eine Trübung eintrat. Nach 1 h konnte das Methansulfonat (Beispiel 11) als weißer Feststoff mit einem Schmelzpunkt von 118-142 °C in einer Ausbeute von 93 % (442 mg) abgesaugt werden.

### Beispiel 12

### N,N-Dimethyl-N-{4-pyridin-1',3',4',5'-tetrahydro-spiro[cyclohexan-1,1'-pyrano[4,3-b]indol]-4-yl}amin

### Beispiel 13

### N,N-Dimethyl-N-{4-pyridin-1',3',4',5'-tetrahydro-spiro[cyclohexan-1,1'-pyrano[4,3-b]indol]-4-yl}amin-citrat

Unter Argon wurde 4-Dimethylamino-4-pyridin-2-yl-cyclohexanon (218 mg, 1 mmol) zusammen mit 2-Benzo[b]thiophen-2-ylethanol (178 mg, 1 mmol) in absolutem Dichlormethan (5 ml) gelöst. Anschließend erfolgte die Zugabe von Methansulfonsäure (3 ml). Der Ansatz wurde 3 d bei RT gerührt. Zur Aufarbeitung wurde das Reaktionsgemisch mit Eis (5 g) und Wasser (30 ml) versetzt. Nach der Neutralisation mit NaHCO₃ (4,4 g, 52 mmol) und der Zugabe von 5N NaOH (1 ml) wurde Dichlormethan (10 ml) hinzugefügt. Die organische Phase wurde abgetrennt und die wäßrige Phase mit Dichlormethan (2 × 30 ml) extrahiert. Die organischen Extrakte wurden nach dem Trocknen eingeengt und der Rückstand (375 mg) chromatographisch an Kieselgel (45 g) mit Ethylacetat/Methanol (10 : 1), (4 : 1) und Methanol getrennt. Dabei wurde der Spiroether (Beispiel 12) in einer Ausbeute von 37 % (143 mg) als farbloser Feststoff mit einem Schmelzpunkt von 155-168 °C isoliert.

Zur Herstellung des Citrats (Beispiel 13) wurde die Base (Beispiel 12) (143 mg, 0,377 mmol) in Ethanol (10 ml) bei 50 °C gelöst und mit Citronensäure (72 mg, 0,377 mmol), gelöst in warmem Ethanol (3 ml), versetzt. Die Reaktionsmischung wurde 2 h bei RT gerührt und auf 5 ml eingeengt. Der dabei ausgefallene Feststoff wurde abgesaugt und mit Ethanol (2 × 1 ml) gewaschen. Das Citrat wurde in einer Ausbeute von 83 % (179 mg) als farbloser Feststoff mit einem Schmelzpunkt von 189-191 °C erhalten.

### Beispiel 14

### N,N-Dimethyl-N-{4-phenyl-1',4'-dihydrospiro[cyclohexan-1,4'-2'H-3',9'-dioxafluoren]-4-yl}ammonium-methansulfonat

Unter Argon wurde 4-Dimethylamino-4-phenyl-cyclohexanon (217 mg, 1 mmol) zusammen mit 2-Benzofuran-2-ylethanol (162,2 mg, 1 mmol) in absolutem Dichlormethan (10 ml) vorgelegt. Anschließend erfolgte die Zugabe von Methansulfonsäuretrimethylsilylester (841,5 mg, 5 mmol). Der Ansatz wurde 24 h bei RT gerührt, erneut Methansulfonsäuretrimethylsilylester (336,6 mg, 2 mmol) zugegeben und weitere 6 h gerührt. Zur Aufarbeitung wurde die klare braune Lösung mit Eis (5 g) versetzt. Nach kurzer Zeit hatte sich ein Feststoff abgesetzt, der abgesaugt und mit Wasser (2 × 10 ml) gewaschen wurde. Das Methansulfonat (Beispiel 14) wurde in einer Ausbeute von 67 % (308 mg) als farbloser Feststoff mit einem Schmelzpunkt von 249-253 °C erhalten.

### Untersuchungen zur Wirksamkeit der erfindungsgemäßen Verbindungen:

### Messung der ORL1-Bindung

Die Cyclohexan-Derivate der allgemeinen Formel I wurden in einem Rezeptorbindungsassay mit ³H-Nociceptin/Orphanin FQ mit Membranen von rekombinanten CHO-ORL1 Zellen untersucht. Dieses Testsystem wurde gemäß der von Ardati et al. (Mol. Pharmacol., 51, 1997, S. 816-824) vorgestellten Methode durchgeführt. Die Konzentration von ³H-Nociceptin/Orphanin FQ betrug bei diesen Versuchen 0.5 nM. Die Bindungsassays wurden mit je 20 µg Membranprotein je 200 µl Ansatz in 50 mM Hepes, pH 7,4, 10 mM MgCl₂ und 1 mM EDTA durchgeführt. Die Bindung an den ORL1-Rezeptor wurde unter Verwendung von je 1 mg WGA-SPA Beads (Amersham-Pharmacia, Freiburg), durch einstündige Inkubation des Ansatzes bei RT und anschliessende Messung im Szintillationscounter Trilux (Wallac, Finnland), bestimmt. Die Affinität wird in Tabelle 1 als nanomolarer Kⱼ-Wert in oder % Inhibition bei c=1 µM angegeben.

Beispielhaft wurde für Beispiel 13 ein Wert von 49% Hemmung bei einer Testkonzentration von 1 µM ermittelt.

### Messung der µ-Bindung

Die Rezeptoraffinität zum humanen µ-Opiatrezeptor wurde in einem homogenen Ansatz in Mikrotiterplatten bestimmt. Hierzu wurden Verdünnungsreihen des jeweils zu prüfenden substituierten spirocyclischen Cyclohexan-Derivates mit einer Rezeptormembranpräparation (15-40 µg Protein pro 250 µl Inkubationsansatz) von CHO-K1-Zellen, welche den humanen µ-Opiatrezeptor exprimieren (RB-HOM-Rezeptormembran-Präparation der Firma NEN, Zaventem, Belgien) in Gegenwart von 1 nmol/l des radioaktiven Liganden [³H]-Naloxon (NET719, Firma NEN, Zaventem, Belgien) sowie von 1 mg WGA-SPA-Beads (Wheat germ agglutinin SPA Beads der Firma Amersham/Pharmacia, Freiburg, Deutschland) in einem Gesamtvolumen von 250 µl für 90 Minuten bei Raumtemperatur inkubiert. Als Inkubationspuffer wurde 50 mmol/l Tris-HCl supplementiert mit 0,05 Gew.-% Natriumazid und mit 0,06 Gew.-% bovinem Serumalbumin verwendet. Zur Bestimmung der unspezifischen Bindung wurde zusätzlich 25 µmol/l Naloxon zugegeben. Nach Beendigung der neunzigminütigen Inkubationszeit wurden die Mikrotiterplatten für 20 Minuten bei 1000 g abzentrifugiert und die Radioaktivität in einem β-Counter (Microbeta-Trilux, Firma PerkinElmer Wallac, Freiburg, Deutschland) vermessen. Es wurde die prozentuale Verdrängung des radioaktiven Liganden aus seiner Bindung zum humanen µ-Opiatrezeptor bei einer Konzentration der Prüfsubstanzen von 1 µmol/l bestimmt und als prozentuale Hemmung (%Hemmung) der spezifischen Bindung angegeben. Teilweise wurden ausgehend von der prozentualen Verdrängung durch unterschiedliche Konzentrationen der zu prüfenden Verbindungen der allgemeinen Formel I IC₅₀ Hemmkonzentrationen berechnet, die eine 50-prozentige Verdrängung des radioaktiven Liganden bewirken. Durch Umrechnung mittels der Cheng-Prusoff-Beziehung wurden Ki-Werte für die Prüfsubstanzen erhalten.

Beispielhaft wurden die folgenden Bindungsdaten bestimmt:

| **Beispiel** | **% Hemmung [1 µM]** |
|---|---|
| **3** | 61 |
| **7** | 54 |
| **4** | 62 |
| **5** | 59 |
| **13** | 78 |
| **14** | 60 |

### Bindungsstelle 2 (BTX-Bindung):

Die Bindungsstelle 2 des Natriumkanals ist die sogenannte Batrachotoxin-(BTX) Bindungsstelle. Als Ligand wurde [³H]-Batrachotoxinin A20 α-Benzoat (10 nM im Ansatz) eingesetzt. Diese Ionen kanal-Partikel (Synaptosomen) wurden aus dem Ratten Cerebrocortex nach Gray und Whittaker (E.G. Gray und V.P. Whittaker (1962) J. Anat. 76, 79-88) angereichert. Als unspezifische Bindung ist die Radioaktivität definiert, die in Gegenwart von Veratridin (0,3 mM im Ansatz) gemessen wird. Inkubation bei 25°C für 120 min. Die Assaybedingungen sind nach der Veröffentlichung von Pauwels, Leysen und Laduron (P.J. Pauwels, J.E. Leysen und P.M. Laduron (1986) Eur. J. Pharmacol. 124, 291-298) durchgeführt worden.

| **Beispiel** | **% Hemmung [10 µM]** |
|---|---|
| **3** | 76 |
| **7** | 82 |
| **8** | 71 |
| **4** | 64 |
| **5** | 74 |
| **9** | 76 |
| **10** | 75 |
| **11** | 74 |
| **13** | 84 |
| **14** | 86 |

### Parenterale Lösung eines erfindungsgemäßen spirocyclischen Cyclohexan-Derivats

38 g eines der erfindungsgemäßen spirocyclischen Cyclohexan-Derivate, hier Beispiel 3, wird in 1 1 Wasser für Injektionszwecke bei Raumtemperatur gelöst und anschließend durch Zugabe von wasserfreier Glukose für Injektionszwecke auf isotone Bedingungen eingestellt.

## Patentansprüche

1. Spirocyclische Cyclohexan-Derivate der allgemeinen Formel I, 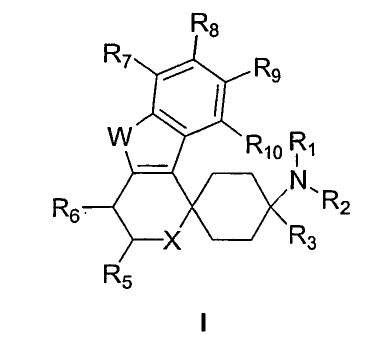 , worin
R¹ und R², unabhängig voneinander für H; CHO; C₁₋₅-Alkyl jeweils gesättigt oder ungesättigt, verzweigt oder unverzweigt, einfach oder mehrfach substituiert oder unsubstituiert; C₃₋₈-Cycloalkyl, jeweils gesättigt oder ungesättigt, einfach oder mehrfach substituiert oder unsubstituiert; oder über C₁₋₃-Alkyl gebundenes Aryl, C₃₋₈-Cycloalkyl oder Heteroaryl, jeweils einfach oder mehrfach substituiert oder unsubstituiert, stehen;
oder die Reste R¹ und R² zusammen für CH₂CH₂OCH₂CH₂, CH₂CH₂NR¹¹ CH₂CH₂ oder (CH₂)₃₋₆ stehen,
wobei R¹¹ H; C₁₋₅-Alkyl, jeweils gesättigt oder ungesättigt, verzweigt oder unverzweigt, einfach oder mehrfach substituiert oder unsubstituiert; C₃₋₈-Cycloalkyl, jeweils gesättigt oder ungesättigt, einfach oder mehrfach substituiert oder unsubstituiert; Aryl-, oder Heteroaryl, jeweils einfach oder mehrfach substituiert oder unsubstituiert; oder über C₁₋₃-Alkyl gebundenes Aryl, C₃₋₈-Cycloalkyl oder Heteroaryl, jeweils einfach oder mehrfach substituiert oder unsubstituiert, bedeutet;
R³ für C₁₋₅-Alkyl, jeweils gesättigt oder ungesättigt, verzweigt oder unverzweigt, einfach oder mehrfach substituiert oder unsubstituiert; C₃₋₈-Cycloalkyl, jeweils gesättigt oder ungesättigt, einfach oder mehrfach substituiert oder unsubstituiert; Aryl oder Heteroaryl, jeweils unsubstituiert oder einfach oder mehrfach substituiert; über C₁₋₃-Alkyl-Gruppe gebundenes Aryl, Heteroaryl oder C₃₋₈-Cycloalkyl, jeweils unsubstituiert oder einfach oder mehrfach substituiert, steht;
W für NR⁴, O oder S steht
und
R⁴ für H; C₁₋₅-Alkyl, gesättigt oder ungesättigt, verzweigt oder unverzweigt, unsubstituiert oder einfach oder mehrfach substituiert; Aryl, oder Heteroaryl, jeweils substituiert oder unsubstituiert; über eine C₁₋₃-Alkyl-Gruppe gebundenes Aryl, Heteroaryl oder Cycloalkyl, jeweils einfach oder mehrfach substituiert oder unsubstituiert; COR¹²; SO₂R¹² steht,
wobei R¹² H; C₁₋₅-Alkyl, jeweils gesättigt oder ungesättigt, verzweigt oder unverzweigt, einfach oder mehrfach substituiert oder unsubstituiert; C₃₋₈-Cycloalkyl, jeweils gesättigt oder ungesättigt, einfach oder mehrfach substituiert oder unsubstituiert; Aryl-, oder Heteroaryl, jeweils einfach oder mehrfach substituiert oder unsubstituiert; oder über C₁₋₃-Alkyl gebundenes Aryl, C₃₋₈-Cycloalkyl oder Heteroaryl, jeweils einfach oder mehrfach substituiert oder unsubstituiert; OR¹³; NR¹⁴R¹⁵ bedeutet;
R⁵ für =O; H; COOR¹³, CONR¹³, OR¹³; C₁₋₅-Alkyl, gesättigt oder ungesättigt, verzweigt oder unverzweigt, unsubstituiert oder einfach oder mehrfach substituiert; C₃₋₈-Cycloalkyl, gesättigt oder ungesättigt, unsubstituiert oder einfach oder mehrfach substituiert; Aryl-, oder Heteroaryl, unsubstituiert oder einfach oder mehrfach substituiert; oder über C₁₋₃-Alkyl gebundenes Aryl, C₃₋₈-Cycloalkyl oder Heteroaryl, unsubstituiert oder einfach oder mehrfach substituiert, steht;
R⁶ für H; F, Cl, NO₂, CF₃, OR¹³, SR¹³, SO₂R¹³, SO₂OR¹³, CN, COOR¹³, NR¹⁴R¹⁵; C₁₋₅-Alkyl, gesättigt oder ungesättigt, verzweigt oder unverzweigt, unsubstituiert oder einfach oder mehrfach substituiert; C₃₋₈-Cycloalkyl, gesättigt oder ungesättigt, unsubstituiert oder einfach oder mehrfach substituiert; Aryl-, oder Heteroaryl, unsubstituiert oder einfach oder mehrfach substituiert; oder über C₁₋₃-Alkyl gebundenes Aryl, C₃₋₈-Cycloalkyl oder Heteroaryl, unsubstituiert oder einfach oder mehrfach substituiert, steht;
oder R⁵ und R⁶ gemeinsam (CH₂)ₙ mit n = 2, 3, 4, 5 oder 6 bedeuten, wobei einzelne Wasserstoffatome auch durch F, Cl, Br, I, NO₂, CF₃, OR¹³, CN oder C₁-₅-Alkyl ersetzt sein können;
R⁷, R⁸, R⁹ und R¹⁰ unabhängig voneinander für H, F, Cl, Br, I, NO₂, CF₃, OR¹³, SR¹³, SO₂R¹³, SO₂OR¹³, CN, COOR¹³, NR¹⁴R¹⁵; C₁₋₅-Alkyl, C₃₋₈-Cycloalkyl, unsubstituiert oder einfach oder mehrfach substituiert; Aryl-, oder Heteroaryl, unsubstituiert oder einfach oder mehrfach substituiert; oder über C₁₋₃-Alkyl gebundenes Aryl, C₃₋₈-Cycloalkyl oder Heteroaryl, unsubstituiert oder einfach oder mehrfach substituiert, stehen;
wobei R¹³ H; C₁₋₅-Alkyl jeweils gesättigt oder ungesättigt, verzweigt oder unverzweigt, unsubstituiert oder einfach oder mehrfach substituiert; C₃₋₈-Cycloalkyl, jeweils gesättigt oder ungesättigt, unsubstituiert oder einfach oder mehrfach substituiert; Aryl-, oder Heteroaryl, unsubstituiert oder einfach oder mehrfach substituiert; oder über C₁₋₃-Alkyl gebundenes Aryl, C₃₋₈-Cycloalkyl oder Heteroaryl, unsubstituiert oder einfach oder mehrfach substituiert, bedeutet;
R¹⁴ und R¹⁵ unabhängig voneinander H; C₁₋₅-Alkyl , jeweils gesättigt oder ungesättigt, verzweigt oder unverzweigt, unsubstituiert oder einfach oder mehrfach substituiert; oder C₃₋₈ -Cycloalkyl, jeweils gesättigt oder ungesättigt, unsubstituiert oder einfach oder mehrfach substituiert; Aryl-, oder Heteroaryl, unsubstituiert oder einfach oder mehrfach substituiert; oder über C₁₋₃-Alkyl gebundenes Aryl, C₃₋₈-Cycloalkyl oder Heteroaryl, unsubstituiert oder einfach oder mehrfach substituiert, bedeuten;
oder R¹⁴ und R¹⁵ zusammen CH₂CH₂OCH₂CH₂, CH₂CH₂NR¹⁶CH₂CH₂ oder (CH₂)₃₋₆ bilden,
wobei R¹⁶ H; C₁₋₅-Alkyl gesättigt oder ungesättigt, verzweigt oder unverzweigt, unsubstituiert oder einfach oder mehrfach substituiert, bedeutet;
X für O, S, SO, SO₂ oder NR¹⁷ steht;
R¹⁷ für H; C₁₋₅-Alkyl, gesättigt oder ungesättigt, verzweigt oder unverzweigt; COR¹² oder SO₂R¹² steht,
in Form des Razemats; der Enantiomere, Diastereomere, Mischungen der Enantiomere oder Diastereomere oder eines einzelnen Enantiomers oder Diastereomers; der Basen und/oder Salze physiologisch verträglicher Säuren oder Kationen.

2. Spirocyclische Cyclohexan-Derivate gemäß Anspruch 1, **dadurch gekennzeichnet, dass**
R¹ und R² unabhängig voneinander für H; C₁₋₈-Alkyl, gesättigt oder ungesättigt, verzweigt oder unverzweigt, einfach oder mehrfach substituiert oder unsubstituiert, stehen;
oder die Reste R¹ und R² zusammen einen Ring bilden und CH₂CH₂OCH₂CH₂, CH₂CH₂NR¹¹CH₂CH₂ oder (CH₂)₃₋₆ bedeuten,
wobei R¹¹ H; C₁-₈-Alkyl, gesättigt oder ungesättigt, verzweigt oder unverzweigt, einfach oder mehrfach substituiert oder unsubstituiert, bedeutet.

3. Spirocyclische Cyclohexan-Derivate gemäß Anspruch 1, **dadurch gekennzeichnet, dass** dass R¹ und R² unabhängig voneinander für CH₃ oder H stehen, wobei R¹ und R² nicht gleichzeitig H bedeuten.

4. Spirocyclische Cyclohexan-Derivate gemäß einem der Ansprüche 1 bis 3, **dadurch gekennzeichnet, dass**
R³ für Cyclopentyl, Cyclohexyl, Phenyl, Benzyl, Naphthyl, Anthracenyl, Thiophenyl, Benzothiophenyl, Furyl, Benzofuranyl, Benzodioxolanyl, Indolyl, Indanyl, Benzodioxanyl, Pyrrolyl, Pyridyl, Pyrimidyl oder Pyrazinyl, jeweils unsubstituiert oder einfach oder mehrfach substituiert; über eine gesättigte, unverzweigte C₁₋₂-Alkyl-Gruppe gebundenen C₅₋₆-Cycloalkyl, Phenyl, Naphthyl, Anthracenyl, Thiophenyl, Benzothiophenyl, Pyridyl, Furyl, Benzofuranyl, Benzodioxolanyl, Indolyl, Indanyl, Benzodioxanyl, Pyrrolyl, Pyrimidyl oder Pyrazinyl, jeweils unsubstituiert oder einfach oder mehrfach substituiert, steht;
insbesondere
R³ Phenyl, Furyl, Thiophenyl, Naphthyl, Benzyl, Benzofuranyl, Indolyl, Indanyl, Benzodioxanyl, Benzodioxolanyl, Pyridyl, Pyrimidyl; Pyrazinyl oder Benzothiophenyl, jeweils unsubstituiert oder einfach oder mehrfach substituiert; über eine gesättigte, unverzweigte C₁₋₂-Alkyl-Gruppe gebundenen Phenyl, Furyl oder Thiophenyl, jeweils unsubstituiert oder einfach oder mehrfach substituiert, bedeutet.

5. Spirocyclische Cyclohexan-Derivate gemäß einem der Ansprüche 1 bis 3, **dadurch gekennzeichnet, dass** R³ für Phenyl, Phenethyl, Thiophenyl, Pyridyl oder Benzyl, jeweils substituiert oder unsubstituiert, steht, besonders bevorzugt für Phenyl.

6. Spirocyclische Cyclohexan-Derivate gemäß einem der Ansprüche 1 bis 3, **dadurch gekennzeichnet, dass** R³ Phenyl bedeutet, unsubstituiert oder mit F, Cl, CN, OCH₃, OCH₂CH₃, CH₃, CF₃ oder OH einfach oder mehrfach substituiert, insbesondere 3-Fluorphenyl und 4-Fluorphenyl.

7. Spirocyclische Cyclohexan-Derivate gemäß einem der Ansprüche 1 bis 6, **dadurch gekennzeichnet, dass** R⁵ für H, C₁₋₅-Alkyl, verzweigt oder unverzweigt, unsubstituiert oder einfach oder mehrfach substituiert oder COOR¹³ steht und R⁶ H oder C₁₋₅-Alkyl bedeutet.

8. Spirocyclische Cyclohexan-Derivate gemäß einem der Ansprüche 1 bis 7, **dadurch gekennzeichnet, dass** R⁷, R⁸, R⁹ und R¹⁰ unabhängig voneinander für H; C₁₋₅-Alkyl, verzweigt oder unverzweigt, unsubstituiert oder ein- oder mehrfach substituiert; F, Cl, Br, I, CF₃, OH, OCH₃, NH₂, COOH, COOCH₃, NHCH₃ oder N(CH₃)₂ oder NO₂ stehen.

9. Spirocyclische Cyclohexan-Derivate gemäß einem der Ansprüche 1 bis 6, **dadurch gekennzeichnet, dass** die Reste R⁵-R¹⁰ H bedeuten.

10. Spirocyclische Cyclohexan-Derivate gemäß einem der Ansprüche 1 bis 9 aus der Gruppe
*N,N*-Dimethyl-*N*-{4-phenyl-1',3',4',5'-tetrahydro-spiro[cyclohexan-1,1'-pyrano[4,3-b]indol]-4-yl}amin
*N*,*N*-Dimethyl-*N*-{4-phenyl-1',3',4',5'-tetrahydro-spiro[cyclohexan-1,1'-pyrano[4,3-b]indol]-4-yl}amin-triflat
*N,N*-Dimethyl-*N*-{4-phenyl-1',3',4',5'-tetrahydro-spiro[cyclohexan-1,1'-pyrano[4,3-b]indol]-4-yl}amin-citrat
*N,N-*Dimethyl-*N*-{4-(4-fluorphenyl)-1',3',4',5'-tetrahydro-spiro[cyclohexan-1,1'-pyrano[4,3-b]indol]-4-yl}ammonium-triflat
*N,N*-Dimethyl-*N*-{4-(3-fluorphenyl)-1',3',4',5'-tetrahydro-spiro[cyclohexan-1,1'-pyrano[4,3-b]indol]-4-yl}ammonium-triflat
*N,N*-Dimethyl-*N*-{4-phenyl-spiro[cyclohexan-1,4'-1',4'-dihydro-2'*H*-3'-oxa-9'-thiafluoren]-4-yl}amin
*N,N*-Dimethyl-*N*-{4-phenyl-spiro[cyclohexan-1,4'-1',4'-dihydro-2'*H*-3'-oxa-9'-thiafluoren]-4-yl}amin-triflat
*N,N*-Dimethyl-*N*-{4-(4-fluorphenyl)-spiro[cyclohexan-1,4'-1',4'-dihydro-2'*H*-3'-oxa-9'-thiafluoren]-4-yl}ammonium-triflat
*N,N*-Dimethyl-*N*-{4-(3-fluorphenyl)-spiro[cyclohexan-1,4'-1',4'-dihydro-2'*H*-3'-oxa-9'-thiafluoren]-4-yl}ammonium-triflat
*N,N*-Dimethyl-N-{4-phenyl-1',4'-dihydrospiro[cyclohexan-1,4'-2'*H*-3',9'-dithiafluoren]-4-yl}ammonium-methansulfonat
*N,N*-Dimethyl-*N*-{4-phenyl-1',4'-dihydrospiro[cyclohexan-1,4'-2'*H*-9'-oxa-3'-thiafluoren]-4-yl}ammonium-methansulfonat
*N,N*-Dimethyl-*N*-{4-pyridin-1',3',4',5'-tetrahydro-spiro[cyclohexan-1,1'-pyrano[4,3-b]indol]-4-yl}amin
*N,N*-Dimethyl-*N*-{4-pyridin-1',3',4',5'-tetrahydro-spiro[cyclohexan-1,1'-pyrano[4,3-b]indol]-4-yl}amin-citrat
*N,N*-Dimethyl-*N*-{4-phenyl-1',4'-dihydrospiro[cyclohexan-1,4'-2'*H*-3',9'-dioxafluoren]-4-yl}ammonium-methansulfonat
in Form des Razemats; der Enantiomere, Diastereomere, Mischungen der Enantiomere oder Diastereomere oder eines einzelnen Enantiomers oder Diastereomers; der Basen und/oder Salze physiologisch verträglicher Säuren oder Kationen.

11. Verfahren zur Herstellung von spirocyclischen Cyclohexanderivaten gemäß einem der Ansprüche 1 bis 10, **dadurch gekennzeichnet, dass** ein Edukt der allgemeinen Formel A 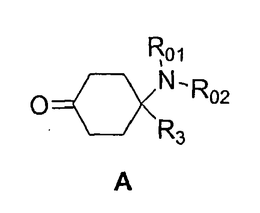 wobei die Reste R⁰¹ und R⁰² die für R² angegebene Bedeutung haben und zusätzlich für eine Schutzgruppe stehen können, unter Zugabe von Säure, oder deren Trimethylsilylester, beispielsweise Trifluormethansulfonsäuretrimethylsilylester, Trifluormethansulfonsäure, Essigsäure, Phosphorsäure, Methansulfonsäure oder Trifluoressigsäure, in einem geeigneten Lösungsmittel, beispielsweise Dichlorethan, Dichlormethan, Chloroform, Acetonitril, Diethylether oder Nitromethan, mit einem Edukt der allgemeinen Formel B 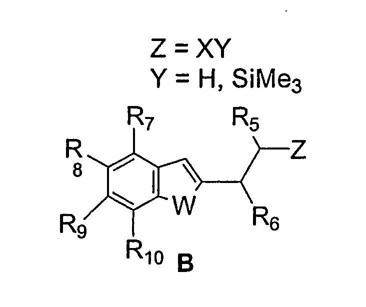 umgesetzt wird, wobei die Reste R¹-R¹⁰ die in Anspruch 1 angegebenen Bedeutungen haben.

12. Verfahren zur Herstellung von spirocyclischen Cyclohexanderivaten gemäß Anspruch 1, bei denen X NR¹⁷ und R¹⁷ COR¹² oder SO₂R¹² bedeutet, **dadurch gekennzeichnet, dass** ein spirocyclisches Cyclohexanderivat, bei dem X NH bedeutet, unter Zugabe von Base, beispielsweise Triethylamin, mit einem Anhydrid oder einem Säurechlorid umgesetzt wird, vorzugsweise unter Mikrowelleneinstrahlung.

13. Verfahren zur Herstellung von spirocyclischen Cyclohexanderivaten gemäß Anspruch 1, bei denen X SO oder SO₂ bedeutet, **dadurch gekennzeichnet, dass** ein spirocyclisches Cyclohexanderivat, bei dem X S bedeutet, mit Hilfe eines Oxidationsmittels, beispielsweise H₂O₂, oxidiert wird.

14. Arzneimittel enthaltend wenigstens ein spirocyclisches Cyclohexan -Derivat gemäß einem der Ansprüche 1 bis 10, gegebenenfalls in Form seines Razemats, der reinen Stereoisomeren, insbesondere Enantiomeren und Diastereomeren, in einem beliebigen Mischungsverhältnis; in Form seiner Säuren oder seiner Basen oder in Form seiner Salze, insbesondere der physiologisch verträglichen Salze oder Salze physiologisch verträglicher Säuren oder Kationen; oder in Form seiner Solvate, insbesondere der Hydrate, sowie gegebenenfalls enthaltend geeignete Zusatz- und/oder Hilfsstoffe und/oder gegebenenfalls weiterer Wirkstoffe.

15. Verwendung eines spirocyclischen Cyclohexan-Derivates gemäß einem der Ansprüche 1 bis 10, gegebenenfalls in Form seines Razemats, der reinen Stereoisomeren, insbesondere Enantiomeren und Diastereomeren, in einem beliebigen Mischungsverhältnis; in Form seiner Säuren oder seiner Basen oder in Form seiner Salze, insbesondere der physiologisch verträglichen Salze oder Salze physiologisch verträglicher Säuren oder Kationen; oder in Form seiner Solvate, insbesondere der Hydrate; zur Herstellung eines Arzneimittels zur Behandlung von Schmerz, insbesondere von akutem, neuropathischem oder chronischem Schmerz.

16. Verwendung eines spirocyclisches Cyclohexan-Derivats gemäß einem der Ansprüche 1 bis 10 zur Herstellung eines Arzneimittels zur Behandlung von Angstzuständen, von Stress und mit Stress verbundenen Syndromen, Depressionen, Epilepsie, Alzheimer Erkrankung, seniler Demenz, allgemeinen kognitiven Dysfunktionen, Lern- und Gedächtnis-Störungen (als Nootropikum), Entzugserscheinungen, Alkohol- und/oder Drogen- und/oder Medikamentenmißbrauch und/oder -abhängigkeit, sexuellen Dysfunktionen, cardiovaskulären Erkrankungen, Hypotension, Hypertension, Tinitus, Pruritus, Migräne, Schwerhörigkeit, mangelnder Darmmotilität, gestörter Nahrungsaufnahme, Anorexie, Fettsucht, lokomotorischen Störungen, Diarrhoe, Kachexie, Harninkontinenz bzw. als Muskelrelaxanz, Antikonvulsivum oder Anesthetikum bzw. zur Coadministration bei Behandlung mit einem opioiden Analgetikum oder mit einem Anesthetikum, als Lokalanesthetikum, zur Diurese oder Antinatriurese, Anxiolyse, zur Modulation der Bewegungsaktivität, zur Modulation der Neurotransmitter-Ausschüttung und Behandlung damit verbundener neurodegenerativer Erkrankungen, zur Behandlung von Entzugserscheinungen und/oder zur Reduzierung des Suchtpotentials von Opioiden.

## Claims

1. Spirocyclic cyclohexane derivatives of the general formula I, 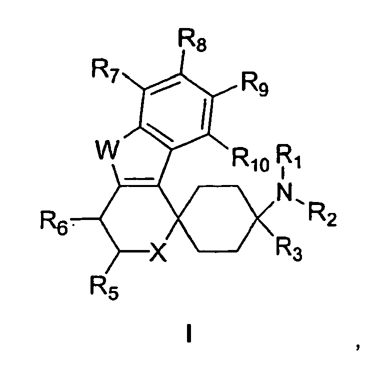 in which
R¹ and R² mutually independently denote H; CHO; C₁₋₅ alkyl in each case saturated or unsaturated, branched or unbranched, mono- or polysubstituted or unsubstituted; C₃₋₈ cycloalkyl, in each case saturated or unsaturated, mono- or polysubstituted or unsubstituted; or aryl, C₃₋₈cycloalkyl or heteroaryl, in each case mono- or polysubstituted or unsubstituted, attached via C₁₋₃ alkyl;
or the residues R¹ and R² together denote CH₂CH₂OCH₂CH₂, CH₂CH₂NR¹¹CH₂CH₂ or (CH₂)₃₋₆,
wherein R¹¹ means H; C₁₋₅ alkyl, in each case saturated or unsaturated, branched or unbranched, mono- or polysubstituted or unsubstituted; C₃₋₈ cycloalkyl, in each case saturated or unsaturated, mono- or polysubstituted or unsubstituted; aryl or heteroaryl, in each case mono- or polysubstituted or unsubstituted; or aryl, C₃₋₈ cycloalkyl or heteroaryl, in each case mono- or polysubstituted or unsubstituted, attached via C₁₋₃ alkyl;
R³ denotes C₁₋₅ alkyl, in each case saturated or unsaturated, branched or unbranched, mono- or polysubstituted or unsubstituted; C₃₋₈ cycloalkyl, in each case saturated or unsaturated, mono- or polysubstituted or unsubstituted; aryl or heteroaryl, in each case unsubstituted or mono- or polysubstituted; aryl, heteroaryl or C₃₋₈ cycloalkyl, in each case unsubstituted or mono- or polysubstituted, attached via C₁₋₃ alkyl group;
W denotes NR⁴, O or S
and
R⁴denotes H; C₁₋₅ alkyl, saturated or unsaturated, branched or unbranched, unsubstituted or mono- or polysubstituted; aryl or heteroaryl, in each case substituted or unsubstituted; aryl, heteroaryl or cycloalkyl, in each case mono- or polysubstituted or unsubstituted, attached via a C₁₋₃ alkyl group; COR¹²; SO₂R¹²,
wherein R¹² means H; C₁₋₅ alkyl, in each case saturated or unsaturated, branched or unbranched, mono- or polysubstituted or unsubstituted; C₃₋₈ cycloalkyl, in each case saturated or unsaturated, mono- or polysubstituted or unsubstituted; aryl or heteroaryl, in each case mono-or polysubstituted or unsubstituted; or aryl, C₃₋₈ cycloalkyl or heteroaryl, in each case mono- or polysubstituted or unsubstituted, attached via C₁₋₃ alkyl; OR¹³; NR¹⁴R¹⁵;
R⁵ denotes =O; H; COOR¹³, CONR¹³, OR¹³; C₁₋₅ alkyl, saturated or unsaturated, branched or unbranched, unsubstituted or mono- or polysubstituted; C₃₋₈ cycloalkyl, saturated or unsaturated, unsubstituted or mono- or polysubstituted; aryl or heteroaryl, unsubstituted or mono-or polysubstituted; or aryl, C₃₋₈ cycloalkyl or heteroaryl, unsubstituted or mono- or polysubstituted, attached via C₁₋₃ alkyl;
R⁶denotes H; F, Cl, NO₂, CF₃, OR¹³, SR¹³, SO₂R¹³, SO₂OR¹³, CN, COOR¹³, NR¹⁴R¹⁵; C₁₋₅ alkyl, saturated or unsaturated, branched or unbranched, unsubstituted or mono- or polysubstituted; C₃₋₈ cycloalkyl, saturated or unsaturated, unsubstituted or mono- or polysubstituted; aryl or heteroaryl, unsubstituted or mono- or polysubstituted; or aryl, C₃₋₈ cycloalkyl or heteroaryl, unsubstituted or mono- or polysubstituted, attached via C₁₋₃ alkyl;
or R⁵ and R⁶ together mean (CH₂)ₙ with n = 2, 3, 4, 5 or 6, wherein individual hydrogen atoms may also be replaced by F, Cl, Br, I, NO₂, CF₃, OR¹³, CN or C₁₋₅ alkyl;
R⁷, R⁸, R⁹ and R¹⁰ mutually independently denote H, F, Cl, Br, I, NO₂, CF₃, OR¹³, SR¹³, SO₂R¹³, SO₂OR¹³ CN, COOR¹³, NR¹⁴R¹⁵; C₁₋₅ alkyl, C₃₋₈ cycloalkyl, unsubstituted or mono- or polysubstituted; aryl or heteroaryl, unsubstituted or mono- or polysubstituted; or aryl, C₃₋₈ cycloalkyl or heteroaryl, unsubstituted or mono- or polysubstituted, attached via C₁₋₃ alkyl;
wherein R¹³ means H; C₁₋₅ alkyl in each case saturated or unsaturated, branched or unbranched, unsubstituted or mono-or polysubstituted; C₃₋₈ cycloalkyl, in each case saturated or unsaturated, unsubstituted or mono- or polysubstituted; aryl or heteroaryl, unsubstituted or mono- or polysubstituted; or aryl, C₃₋₈ cycloalkyl or heteroaryl, unsubstituted or mono- or polysubstituted, attached via C₁₋₃ alkyl;
R¹⁴ and R¹⁵ mutually independently mean H; C₁₋₅ alkyl, in each case saturated or unsaturated, branched or unbranched, unsubstituted or mono- or polysubstituted; or C₃₋₈ cycloalkyl, in each case saturated or unsaturated, unsubstituted or mono- or polysubstituted; aryl or heteroaryl, unsubstituted or mono- or polysubstituted; or aryl, C₃₋₈ cycloalkyl or heteroaryl, unsubstituted or mono- or polysubstituted, attached via C₁₋₃ alkyl;
or R¹⁴ and R¹⁵ together form CH₂CH₂OCH₂CH₂, CH₂CH₂NR¹⁶CH₂CH₂ or (CH₂)₃₋₆, wherein R¹⁶ means H; C₁₋₅ alkyl, saturated or unsaturated, branched or unbranched, unsubstituted or mono- or polysubstituted;
X denotes O, S, SO, SO₂ or NR¹⁷;
R¹⁷ denotes H; C₁₋₅ alkyl, saturated or unsaturated, branched or unbranched; COR¹² or SO₂R¹²,
in the form of the racemate; the enantiomers, diastereomers, mixtures of the enantiomers or diastereomers or of an individual enantiomer or diastereomer; the bases and/or salts of physiologically acceptable acids or cations.

2. Spirocyclic cyclohexane derivatives according to claim 1, **characterised in that**
R¹ and R² mutually independently denote H; C₁₋₈ alkyl, saturated or unsaturated, branched or unbranched, mono- or polysubstituted or unsubstituted;
or the residues R¹ and R² together form a ring and mean CH₂CH₂OCH₂CH₂, CH₂CH₂NR¹¹CH₂CH₂, or (CH₂)₃₋₆,
wherein R¹¹ means H; C₁₋₈ alkyl, saturated or unsaturated, branched or unbranched, mono- or polysubstituted or unsubstituted.

3. Spirocyclic cyclohexane derivatives according to claim 1, **characterised in that** R¹ and R² mutually independently denote CH₃ or H, wherein R¹ and R² do not simultaneously mean H.

4. Spirocyclic cyclohexane derivatives according to any one of claims 1 to 3, **characterised in that**
R³ denotes cyclopentyl, cyclohexyl, phenyl, benzyl, naphthyl, anthracenyl, thiophenyl, benzothiophenyl, furyl, benzofuranyl, benzodioxolanyl, indolyl, indanyl, benzodioxanyl, pyrrolyl, pyridyl, pyrimidyl or pyrazinyl, in each case unsubstituted or mono- or polysubstituted; C₅₋₆ cycloalkyl attached via a saturated, unbranched C₁₋₂ alkyl group, phenyl, naphthyl, anthracenyl, thiophenyl, benzothiophenyl, pyridyl, furyl, benzofuranyl, benzodioxolanyl, indolyl, indanyl, benzodioxanyl, pyrrolyl, pyrimidyl or pyrazinyl, in each case unsubstituted or mono- or polysubstituted;
in particular
R³ means phenyl, furyl, thiophenyl, naphthyl, benzyl, benzofuranyl, indolyl, indanyl, benzodioxanyl, benzodioxolanyl, pyridyl, pyrimidyl, pyrazinyl or benzothiophenyl, in each case unsubstituted or mono- or polysubstituted; phenyl, furyl or thiophenyl, in each case unsubstituted or mono- or polysubstituted, attached via a saturated, unbranched C₁₋₂ alkyl group.

5. Spirocyclic cyclohexane derivatives according to any one of claims 1 to 3, **characterised in that** R³ denotes phenyl, phenethyl, thiophenyl, pyridyl or benzyl, in each case substituted or unsubstituted, particularly preferably phenyl.

6. Spirocyclic cyclohexane derivatives according to any one of claims 1 to 3, **characterised in that** R³ means phenyl, unsubstituted or mono- or polysubstituted with F, Cl, CN, OCH₃, OCH₂CH₃, CH₃, CF₃ or OH, in particular 3-fluorophenyl and 4-fluorophenyl.

7. Spirocyclic cyclohexane derivatives according to any one of claims 1 to 6, **characterised in that** R⁵ denotes H, C₁₋₅ alkyl, branched or unbranched, unsubstituted or mono- or polysubstituted or COOR¹³ and R⁶ means H or C₁₋₅ alkyl.

8. Spirocyclic cyclohexane derivatives according to any one of claims 1 to 7, **characterised in that** R⁷ , R⁸, R⁹ and R¹⁰ mutually independently denote H; C₁₋₅ alkyl, branched or unbranched, unsubstituted or mono-or polysubstituted; F, Cl, Br, I, CF₃, OH, OCH₃, NH₂, COOH, COOCH₃, NHCH₃ or N(CH₃)₂ or NO₂.

9. Spirocyclic cyclohexane derivatives according to any one of claims 1 to 6, **characterised in that** the residues R⁵-R¹⁰ mean H.

10. Spirocyclic cyclohexane derivatives according to any one of claims 1 to 9 from the group
*N,N*-dimethyl-*N*-{4-phenyl-1',3',4',5'-tetrahydrospiro[cyclohexane-1,1'-pyrano[4,3b]indol]-4-yl}amine
*N,N*-dimethyl-*N*-{4-phenyl-1',3',4',5'-tetrahydrospiro[cyclohexane-1,1'-pyrano[4,3b]indol]-4-yl}amine triflate
*N,N*-dimethyl-*N*-{4-phenyl-1',3',4',5'-tetrahydrospiro[cyclohexane-1,1'-pyrano[4,3b]indol]-4-yl}amine citrate
*N,N*-dimethyl-*N*-{4-(4-fluorophenyl)-1',3',4',5,-tetrahydrospiro[cyclohexane-1,1'-pyrano[4,3b]indol]-4-yl}ammonium triflate
*N,N*-dimethyl-*N*-{4-(3-fluorophenyl)-1',3',4',5'-tetrahydrospiro[cyclohexane-1,1'-pyrano[4,3b]indol]-4-yl}ammonium triflate
*N,N*-dimethyl-*N*-{4-phenylspiro[cyclohexane-1,4'-1,4'-dihydro-2'*H*-3'-oxa-9'-thiafluoren]-4-yl}amine
*N,N*-dimethyl-*N*-{4-phenylspiro[cyclohexane-1,4'-1,4'-dihydro-2'*H*-3'-oxa-9'-thiafluoren]-4-yl}amine triflate
*N,N*-dimethyl-*N*-{4-(4-fluorophenyl)-spiro[cyclohexane-1,4'-1,4'-dihydro-2'*H*-3'-oxa-9'-thiafluoren]-4-yl}ammonium triflate
*N,N*-dimethyl-*N*-{4-(3-fluorophenyl)-spiro[cyclohexane-1,4'-1,4'-dihydro-2'*H*-3'-oxa-9'-thiafluoren]-4-yl}ammonium triflate
*N,N*-dimethyl-*N*-{4-phenyl-1',4'-dihydrospiro[cyclohexane-1,4'-2'*H*-3',9'-dithiafluoren]-4-yl}ammonium methanesulfonate
*N,N*-dimethyl-*N*-{4-phenyl-1',4'-dihydrospiro[cyclohexane-1,4'-2'*H*-9'-oxa-3'-thiafluoren]-4-yl}ammonium methanesulfonate
*N,N*-dimethyl-*N*-{4-pyridine-1',3',4',5'-tetrahydrospiro[cyclohexane-1,1'-pyrano[4,3b]indol]-4-yl}amine
*N,N*-dimethyl-*N*-{4-pyridine-1',3',4',5'-tetrahydrospiro[cyclohexane-1,1'-pyrano[4,3b]indol]-4-yl}amine citrate
*N,N*-dimethyl-*N*-{4-phenyl-1',4'-dihydrospiro[cyclohexane-1,4'-2'*H*-3',9'-dioxafluoren]-4-yl}ammonium methanesulfonate
in the form of the racemate; the enantiomers, diastereomers, mixtures of the enantiomers or diastereomers or of an individual enantiomer or diastereomer; the bases and/or salts of physiologically acceptable acids or cations.

11. A method for producing spirocyclic cyclohexane derivatives according to any one of claims 1 to 10, **characterised in that** an educt of the general formula A 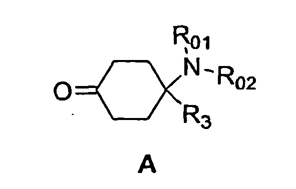 wherein the residues R⁰¹ and R⁰² have the meaning stated for R² and may additionally denote a protective group, is reacted with addition of acid, or the trimethylsilyl ester thereof, for example trifluoromethanesulfonic acid trimethylsilyl ester, trifluoromethanesulfonic acid, acetic acid, phosphoric acid, methanesulfonic acid or trifluoroacetic acid, in a suitable solvent, for example dichloroethane, dichloromethane, chloroform, acetonitrile, diethyl ether or nitromethane, with an educt of the general formula B 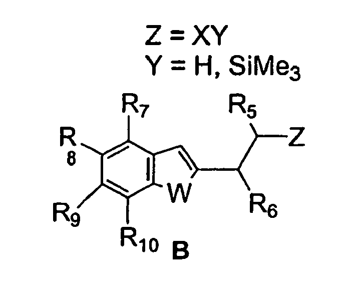 wherein the residues R¹-R¹⁰ have the meanings stated in claim 1.

12. A method for producing spirocyclic cyclohexane derivatives according to claim 1, in which X means NR¹⁷ and R¹⁷ means COR¹² or SO₂R¹², **characterised in that** a spirocyclic cyclohexane derivative, in which X means NH, is reacted with addition of base, for example triethylamine, with an anhydride or an acid chloride, preferably with microwave irradiation.

13. A method for producing spirocyclic cyclohexane derivatives according to claim 1, in which X means SO or SO₂, **characterised in that** a spirocyclic cyclohexane derivative in which X means S is oxidised with the assistance of an oxidising agent, for example H₂O₂.

14. A medicament containing at least one spirocyclic cyclohexane derivative according to any one of claims 1 to 10, optionally in the form the racemate thereof, the pure stereoisomers, in particular enantiomers and diastereomers, in any desired mixing ratio; in the form of the acids or bases thereof or in the form of the salts thereof, in particular the physiologically acceptable salts or salts of physiologically acceptable acids or cations; or in the form of the solvates thereof, in particular the hydrates, and optionally containing suitable additives and/or auxiliary substances and/or optionally further active ingredients.

15. Use of a spirocyclic cyclohexane derivative according to any one of claims 1 to 10, optionally in the form of the racemate thereof, the pure stereoisomers, in particular enantiomers and diastereomers, in any desired mixing ratio; in the form of the acids or bases thereof or in the form of the salts thereof, in particular the physiologically acceptable salts or salts of physiologically acceptable acids or cations; or in the form of the solvates thereof, in particular the hydrates; for producing a medicament for treating pain, in particular acute, neuropathic or chronic pain.

16. Use of a spirocyclic cyclohexane derivative according to any one of claims 1 to 10 for producing a medicament for treating anxiety states, stress and syndromes associated with stress, depression, epilepsy, Alzheimer's disease, senile dementia, general cognitive dysfunction, learning and memory disorders (as a nootropic), withdrawal symptoms, abuse and/or dependency on alcohol and/or drugs and/or medicines, sexual dysfunction, cardiovascular diseases, hypotension, hypertension, tinnitus, pruritus, migraine, hardness of hearing, insufficient intestinal motility, disturbed food intake, anorexia, obesity, locomotor disorders, diarrhoea, cachexia, urinary incontinence or as a muscle relaxant, anticonvulsive or anaesthetic or for coadministration on treatment with an opioid analgesic or with an anaesthetic, as a local anaesthetic, for diuresis or antinatriuresis, anxiolysis, for modulating locomotor activity, for modulating neurotransmitter release and treating neurodegenerative diseases associated therewith, for treating withdrawal symptoms and/or for reducing the addictive potential of opioids.

## Revendications

1. Dérivés spirocycliques de cyclohexane de formule générale I, 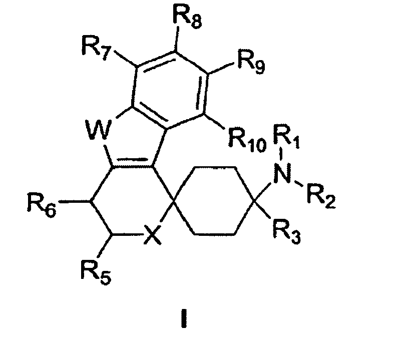 où
R₁ et R₂, représentent, indépendamment l'un de l'autre, H ; CHO; C₁₋₅-alkyle à chaque fois saturé ou insaturé, ramifié ou non ramifié, monosubstitué, polysubstitué ou non substitué ; C₃₋₈-cycloalkyle, à chaque fois saturé ou insaturé, monosubstitué, polysubstitué ou non substitué ; ou aryle, C₃₋₈- cycloalkyle ou hétéroaryle, à chaque fois monosubstitué, polysubstitué ou non substitué, lié par C₁₋₃-alkyle ;
ou les radicaux R₁ et R₂ représentent, ensemble, CH₂CH₂OCH₂CH₂, CH₂CH₂NR¹¹CH₂CH₂ ou (CH₂)₃₋₆,
où
R¹¹ signifie H ; C₁₋₅-alkyle, à chaque fois saturé ou insaturé, ramifié ou non ramifié, monosubstitué, polysubstitué ou non substitué ; C₃₋₈-cycloalkyle, à chaque fois saturé ou insaturé, monosubstitué, polysubstitué ou non substitué ; aryle ou hétéroaryle, à chaque fois monosubstitué, polysubstitué ou non substitué ; ou aryle, C₃₋₈ -cycloalkyle ou hétéroaryle, à chaque fois monosubstitué, polysubstitué ou non substitué, lié par C₁₋₃-alkyle ;
R₃ représente C₁₋₅-alkyle, à chaque fois saturé ou insaturé, ramifié ou non ramifié, monosubstitué, polysubstitué ou non substitué ; C₃₋₈-cycloalkyle, à chaque fois saturé ou insaturé, monosubstitué, polysubstitué ou non substitué ; aryle ou hétéroaryle, à chaque fois non substitué, monosubstitué ou polysubstitué ; aryle, hétéroaryle ou C₃₋₈-cycloalkyle, à chaque fois non substitué, monosubstitué ou polysubstitué, lié par C₁₋₃-alkyle ;
W représente NR⁴, O ou S et
R⁴ représente H ; C₁₋₅-alkyle, saturé ou insaturé, ramifié ou non ramifié, non substitué, monosubstitué ou polysubstitué ; aryle ou hétéroaryle, à chaque fois substitué ou non substitué ; aryle, hétéroaryle ou cycloalkyle, à chaque fois monosubstitué, polysubstitué ou non substitué, lié par un groupe C₁₋₃-alkyle ; COR¹² ; SO₂R¹²,
où
R¹² signifie H ; C₁₋₅-alkyle, à chaque fois saturé ou insaturé, ramifié ou non ramifié, monosubstitué, polysubstitué ou non substitué ; C₃₋₈-cycloalkyle, à chaque fois saturé ou insaturé, monosubstitué, polysubstitué ou non substitué ; aryle ou hétéroaryle, à chaque fois monosubstitué, polysubstitué ou non substitué ; ou aryle, C₃₋₈- cycloalkyle ou hétéroaryle, à chaque fois monosubstitué, polysubstitué ou non substitué, lié par C₁₋₃-alkyle ; OR¹³ ; NR¹⁴R¹⁵ ;
R₅ représente =O ; H ; COOR¹³, CONR¹³, OR¹³ ; C₁₋₅- alkyle, saturé ou insaturé, ramifié ou non ramifié, non substitué, monosubstitué ou polysubstitué ; C₃₋₈-cycloalkyle, saturé ou insaturé, non substitué, monosubstitué ou polysubstitué ; aryle ou hétéroaryle, non substitué, monosubstitué ou polysubstitué ; ou aryle, C₃₋₈-cycloalkyle ou hétéroaryle, non substitué, monosubstitué ou polysubstitué, lié par C₁₋₃-alkyle ;
R₆ représente H ; F, Cl, NO₂, CF₃, OR¹³, SR¹³, SO₂R¹³, SO₂OR¹³, CN, COOR¹³, NR¹⁴R¹⁵ ; C₁₋₅-alkyle, saturé ou insaturé, ramifié ou non ramifié, non substitué, monosubstitué ou polysubstitué ; C₃₋₈-cycloalkyle, saturé ou insaturé, non substitué, monosubstitué ou polysubstitué ; aryle ou hétéroaryle, non substitué, monosubstitué ou polysubstitué ; ou aryle, C₃₋₈-cycloalkyle ou hétéroaryle, non substitué, monosubstitué ou polysubstitué, lié par C₁₋₃-alkyle ;
ou R₅ et R₆ signifient ensemble (CH₂)ₙ avec n = 2, 3, 4, 5 ou 6, où différents atomes d'hydrogène peuvent également être remplacés par F, Cl, Br, I, NO₂, CF₃, OR¹³, CN ou C₁₋₅-alkyle ;
R₇, R₈, R₉ et R₁₀ représentent, indépendamment l'un de l'autre, H, F, Cl, Br, I, NO₂, CF₃, OR¹³, SR¹³, SO₂R¹³, SO₂OR¹³, CN, COOR¹³, NR¹⁴R¹⁵ C₁₋₅- alkyle, C₃₋₈-cycloalkyle, non substitué, monosubstitué ou polysubstitué ; aryle ou hétéroaryle, non substitué, monosubstitué ou polysubstitué ; ou aryle, C₃₋₈-cycloalkyle ou hétéroaryle, non substitué, monosubstitué ou polysubstitué, lié par C₁₋₃-alkyle ;
où
R¹³ signifie H ; C₁₋₅-alkyle, à chaque fois saturé ou insaturé, ramifié ou non ramifié, non substitué, monosubstitué ou polysubstitué ; C₃₋₈-cycloalkyle, à chaque fois saturé ou insaturé, non substitué, monosubstitué ou polysubstitué ; aryle ou hétéroaryle, non substitué, monosubstitué ou polysubstitué ; ou aryle, C₃₋₈-cycloalkyle ou hétéroaryle, non substitué, monosubstitué ou polysubstitué, lié par C₁₋₃-alkyle ;
R¹⁴ et R¹⁵ signifient, indépendamment l'un de l'autre H ; C₁₋₅-alkyle, à chaque fois saturé ou insaturé, ramifié ou non ramifié, non substitué, monosubstitué ou polysubstitué ; ou C₃₋₈-cycloalkyle, à chaque fois saturé ou insaturé, non substitué, monosubstitué ou polysubstitué ; aryle ou hétéroaryle, non substitué, monosubstitué ou polysubstitué ; ou aryle, C₃₋₈-cycloalkyle ou hétéroaryle, non substitué, monosubstitué ou polysubstitué, lié par C₁₋₃-alkyle ; ou R¹⁴ et R¹⁵ forment, ensemble, CH₂CH₂OCH₂CH₂, CH₂CH₂NR¹⁶CH₂CH₂ ou (CH₂)₃₋₆,
où R¹⁶ signifie H ; C₁₋₅-alkyle saturé ou insaturé, ramifié ou non ramifié, non substitué, monosubstitué ou polysubstitué ;
X représente 0, S, SO, SO₂ ou NR¹⁷ ;
R¹⁷ représente H ; C₁₋₅-alkyle, saturé ou insaturé, ramifié ou non ramifié ; COR¹² ou SO₂R¹²,
sous forme du racémate ; des énantiomères, diastéréo-isomères, mélanges des énantiomères ou diastéréo-isomères ou d'un seul énantiomère ou diastéréo-isomère ; des bases et/ou des sels des acides ou cations physiologiquement acceptables.

2. Dérivés spirocycliques de cyclohexane selon la revendication 1, **caractérisés en ce que**
R₁ et R₂ représentent, indépendamment l'un de l'autre H ; C₁₋₈-alkyle saturé ou insaturé, ramifié ou non
où ramifié, monosubstitué, polysubstitué ou non substitué ; ou les radicaux R₁ et R₂ forment ensemble un cycle et représentent CH₂CH₂OCH₂CH₂, CH₂CH₂NR¹¹CH₂CH₂ ou (CH₂)₃₋₆,
R¹¹ signifie H ; C₁₋₈-alkyle saturé ou insaturé, ramifié ou non ramifié, monosubstitué, polysubstitué ou non substitué.

3. Dérivés spirocycliques de cyclohexane selon la revendication 1, **caractérisés en ce que** R₁ et R₂ représentent, indépendamment l'un de l'autre, CH₃ ou H, où R₁ et R₂ ne signifient pas simultanément H.

4. Dérivés spirocycliques de cyclohexane selon l'une quelconque des revendications 1 à 3, **caractérisés en ce que**
R₃ représente cyclopentyle, cyclohexyle, phényle, benzyle, naphtyle, anthracényle, thiophényle, benzothiophényle, furyle, benzofurannyle, benzodioxolanyle, indolyle, indanyle, benzodioxanyle, pyrrolyle, pyridyle, pyrimidyle ou pyrazinyle, à chaque fois non substitué, monosubstitué ou polysubstitué ; C₅₋₆-cycloalkyle, phényle, naphtyle, anthracényle, thiophényle, benzothiophényle, pyridyle, furyle, benzofurannyle, benzodioxolanyle, indolyle, indanyle, benzodioxanyle, pyrrolyle, pyrimidyle ou pyrazinyle, à chaque fois non substitué, monosubstitué ou polysubstitué, lié par un groupe C₁₋₂-alkyle saturé non ramifié ;
en particulier
R₃ signifie phényle, furyle, thiophényle, naphtyle, benzyle, benzofurannyle, indolyle, indanyle, benzodioxanyle, benzodioxolanyle, pyridyle, pyrimidyle, pyrazinyle ou benzothiophényle, à chaque fois non substitué, monosubstitué ou polysubstitué ; phényle, furyle ou thiophényle, à
chaque fois non substitué, monosubstitué ou polysubstitué, lié par un groupe C₁₋₂-alkyle saturé non ramifié.

5. Dérivés spirocycliques de cyclohexane selon l'une quelconque des revendications 1 à 3, **caractérisés en ce que** R₃ représente phényle, phénéthyle, thiophényle, pyridyle ou benzyle, à chaque fois substitué ou non substitué, de manière particulièrement préférée phényle.

6. Dérivés spirocycliques de cyclohexane selon l'une quelconque des revendications 1 à 3, **caractérisés en ce que** R₃ signifie phényle, non substitué ou monosubstitué ou polysubstitué par F, Cl, CN, OCH₃, OCH₂CH₃, CH₃, CF₃ ou OH, en particulier 3-fluorophényle et 4-fluorophényle.

7. Dérivés spirocycliques de cyclohexane selon l'une quelconque des revendications 1 à 6, **caractérisés en ce que** R₅ représente H, C₁₋₅-alkyle, ramifié ou non ramifié, non substitué ou monosubstitué ou polysubstitué ou COOR¹³ et R₆ signifie H ou C₁₋₅-alkyle.

8. Dérivés spirocycliques de cyclohexane selon l'une quelconque des revendications 1 à 7, **caractérisés en ce que** R₇, R₈, R₉ et R₁₀ représentent, indépendamment l'un de l'autre, H ; C₁₋₅-alkyle, ramifié ou non ramifié, non substitué, monosubstitué ou polysubstitué ; F, Cl, Br, I, CF₃, OH, OCH₃, NH₂, COOH, COOCH₃, NHCH₃ ou N(CH₃)₂ ou NO₂.

9. Dérivés spirocycliques de cyclohexane selon l'une quelconque des revendications 1 à 6, **caractérisés en ce que** les radicaux R₅-R₁₀ signifient H.

10. Dérivés spirocycliques de cyclohexane selon l'une quelconque des revendications 1 à 9 du groupe
N,N-diméthyl-N-{4-phényl-1',3',4',5'-tétrahydrospiro[cyclohexane-1,1'-pyrano[4,3-b]indol]-4-yl}amine
N,N-diméthyl-N-{4-phényl-l',3',4',5'-tétrahydrospiro[cyclohexane-1,1'-pyrano[4,3-b]indol]-4-yl}amine-triflate
N,N-diméthyl-N-{4-phényl-1',3',4',5'-tétrahydrospiro[cyclohexane-1,1'-pyrano[4,3-b]indol]-4-yl}amine-citrate Triflate de N,N-diméthyl-N-{4-(4-fluorophényl)-1',3',4',5'-tétrahydrospiro[cyclohexane-1,1'-pyrano[4,3-b]indol]-4-yl}ammonium Triflate de N,N-diméthyl-N-{4-(3-fluorophényl)-1',3',4',5'-tétrahydrospiro[cyclohexane-1,1'-pyrano[4,3-b]indol]-4-yl}ammonium
N,N-diméthyl-N-{4-phénylspiro[cyclohexane-1,4'-1',4'-dihydro-2'H-3'-oxa-9'-thiafluorén]-4-yl}amine N,N-diméthyl-N-{4-phénylspiro[cyclohexane-1,4'-1',4'-dihydro-2'H-3'-oxa-9'-thiafluorén]-4-yl}amine-triflate Triflate de N,N-diméthyl-N-{4-(4-fluorophényl)-spiro[cyclohexane-1,4'-1',4'-dihydro-2'H-3'-oxa-9'-thiafluorén]-4-yl}ammonium Triflate de N,N-diméthyl-N-{4-(3-fluorophényl)-spiro[cyclohexane-1,4'-1',4'-dihydro-2'H-3'-oxa-9'-thiafluorén]-4-yl}ammonium Méthanesulfonate de N,N-diméthyl-N-{4-phényl-1',4'-dihydrospiro[cyclohexane-1,4'-2'H-3',9'-dithiafluorén]-4-yl}ammonium Méthanesulfonate de N,N-diméthyl-N-{4-phényl-1',4'-dihydrospiro[cyclohexane-1,4'-2'H-9'-oxa-3'-thiafluorén]-4-yl}ammonium
N,N-diméthyl-N-{4-pyridine-1',3',4',5'-tétrahydrospiro[cyclohexane-1,1'-pyrano[4,3-b]indol]-4-yl}amine
N,N-diméthyl-N-{4-pyridine-1',3',4',5'-tétrahydrospiro[cyclohexane-1,1'-pyrano[4,3-b]indol]-4-yl}amine-citrate Méthanesulfonate de N,N-diméthyl-N-{4-phényl-1',4'-dihydrospiro[cyclohexane-1,4'-2'H-3',9'-dioxafluorén]-4-yl}ammonium
sous forme du racémate ; des énantiomères, diastéréo-isomères, mélanges des énantiomères ou diastéréo-isomères ou d'un seul énantiomère ou diastéréo-isomère; des bases et/ou des sels des acides ou cations physiologiquement acceptables.

11. Procédé pour la préparation de dérivés spirocycliques de cyclohexane selon l'une quelconque des revendications 1 à 10, **caractérisé en ce qu'**on transforme un produit de départ de formule générale A 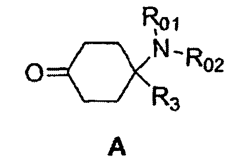 où les radicaux R₀₁ et R₀₂ présentent la signification indiquée pour R₂ et peuvent en outre représenter un groupe de protection, avec addition d'acide, ou de son ester de triméthylsilyle, par exemple l'ester triméthylsilylique de l'acide trifluorométhanesulfonique, l'acide trifluorométhanesulfonique, l'acide acétique, l'acide phosphorique, l'acide méthanesulfonique ou l'acide trifluoroacétique, dans un solvant approprié, par exemple le dichloroéthane, le dichlorométhane, le chloroforme, l'acétonitrile, le diéthyléther ou le nitrométhane, avec un produit de départ de formule générale B 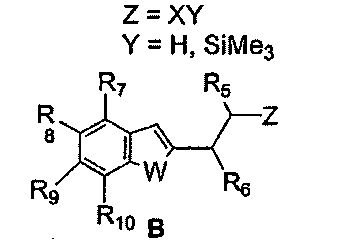 les radicaux R₁-R₁₀ ayant les significations indiquées dans la revendication 1.

12. Procédé pour la préparation de dérivés spirocycliques de cyclohexane selon la revendication 1, dans lesquels X signifie NR¹⁷ et R¹⁷ signifie COR¹² ou SO₂R¹², **caractérisé en ce qu'**on transforme un dérivé spirocyclique de cyclohexane, dans lequel X signifie NH, avec addition d'une base, par exemple la triéthylamine, avec un anhydride ou un chlorure d'acide, de préférence sous un rayonnement de micro-ondes.

13. Procédé pour la préparation de dérivés spirocycliques de cyclohexane selon la revendication 1, dans lesquels X signifie SO ou SO₂, **caractérisé en ce qu'**on oxyde un dérivé spirocyclique de cyclohexane, dans lequel X signifie S, à l'aide d'un oxydant, par exemple H₂O₂.

14. Médicament contenant au moins un dérivé spirocyclique de cyclohexane selon l'une quelconque des revendications 1 à 10, le cas échéant sous forme de son racémate, des stéréo-isomères purs, en particulier des énantiomères et des diastéréo-isomères, dans un rapport de mélange quelconque ; sous forme de ses acides ou de ses bases ou sous forme de ses sels, en particulier les sels physiologiquement acceptables ou les sels d'acides ou de cations physiologiquement acceptables ; ou sous forme de ses solvates, en particulier des hydrates, et contenant le cas échéant des additifs et/ou adjuvants appropriés et/ou le cas échéant d'autres substances actives.

15. Utilisation d'un dérivé spirocyclique de cyclohexane selon l'une quelconque des revendications 1 à 10, le cas échéant sous forme de son racémate, des stéréo-isomères purs, en particulier des énantiomères et des diastéréo-isomères, dans un rapport de mélange quelconque ; sous forme de ses acides ou de ses bases ou sous forme de ses sels, en particulier les sels physiologiquement acceptables ou les sels d'acides ou de cations physiologiquement acceptables ; ou sous forme de ses solvates, en particulier des hydrates pour la préparation d'un médicament destiné au traitement de la douleur, en particulier de la douleur aiguë, neuropathique ou chronique.

16. Utilisation d'un dérivé spirocyclique du cyclohexane selon l'une quelconque des revendications 1 à 10 pour la préparation d'un médicament destiné au traitement des états anxieux, du stress et des syndromes associés au stress, des dépressions, de l'épilepsie, de la maladie d'Alzheimer, de la démence sénile, des dysfonctionnements cognitifs généraux, des troubles de l'apprentissage et de la mémoire (comme nootropique), des symptômes de manque, de l'abus et/ou de la dépendance de l'alcool et/ou des drogues et/ou des médicaments, des dysfonctionnements sexuels, des maladies cardiovasculaires, de l'hypotension, de l'hypertension, des acouphènes, du prurit, de la migraine, de la surdité, d'une déficience de la motilité intestinale, de troubles de l'ingestion alimentaire, de l'anorexie, de la boulimie, des troubles locomoteurs, de la diarrhée, de la cachexie, de l'incontinence urinaire ou comme relaxant musculaire, comme anticonvulsif ou anesthésique ou pour la co-administration lors du traitement avec un analgésique opioïde ou avec un anesthésique, comme anesthésique local, pour la diurèse ou l'antinatriurèse, pour l'anxiolyse, pour la modulation de l'activité des mouvements, pour la modulation de l'excrétion des neurotransmetteurs et le traitement des maladies de neurodégénérescence qui y sont liées, pour le traitement de syndromes de manque et/ou pour la réduction du potentiel de besoin d'opioïdes.
